(19) Europäisches Patentamt
European Patent Office
Office européen des brevets

(11) **EP 1 364 068 B1**

(12) **EUROPÄISCHE PATENTSCHRIFT**

(45) Veröffentlichungstag und Bekanntmachung des Hinweises auf die Patenterteilung:
**19.11.2008 Patentblatt 2008/47**

(21) Anmeldenummer: **02722043.3**

(22) Anmeldetag: **04.02.2002**

(51) Int Cl.:
***C12Q 1/68*** (2006.01)

(86) Internationale Anmeldenummer:
**PCT/EP2002/001126**

(87) Internationale Veröffentlichungsnummer:
**WO 2002/064822 (22.08.2002 Gazette 2002/34)**

(54) **VERFAHREN UND KIT ZUR TIERARTSPEZIFISCHEN DNA-IDENTIFIKATION IN EINER PROBE**

METHOD AND KIT FOR ANIMAL SPECIES-SPECIFIC DNA IDENTIFICATION OF A SAMPLE

PROCEDE ET KIT PERMETTANT L'IDENTIFICATION D'ADN SPECIFIQUE DES ESPECES ANIMALES DANS UN ECHANTILLON

(84) Benannte Vertragsstaaten:
**AT BE CH CY DE DK ES FI FR GB GR IE IT LI LU MC NL PT SE TR**

(30) Priorität: **05.02.2001 DE 10105056**

(43) Veröffentlichungstag der Anmeldung:
**26.11.2003 Patentblatt 2003/48**

(73) Patentinhaber: **Congen Biotechnologie GmbH 13125 Berlin (DE)**

(72) Erfinder:
• **KUHN, Matthias,
Congen Biotechnologie GmbH
13125 Berlin (DE)**
• **MERGEMEIER, Steffen,
Congen Biotechnologie GmbH
13125 Berlin (DE)**

(74) Vertreter: **Hartz, Nikolai et al
Wächtershäuser & Hartz
Patentanwälte
Weinstrasse 8
80333 München (DE)**

(56) Entgegenhaltungen:
**DE-A- 19 629 166        US-A- 6 015 664**

• BARTLETT S E ET AL: "FINS FORENSICALLY INFORMATIVE NUCLEOTIDE SEQUENCING A PROCEDURE FOR IDENTIFYING THE ANIMAL ORIGIN OF BIOLOGICAL SPECIMENS" BIOTECHNIQUES, Bd. 12, Nr. 3, 1992, Seiten 408-411, XP002234795 ISSN: 0736-6205
• IRWIN D M ET AL: "EVOLUTION OF THE CYTOCHROME BETA GENE OF MAMMALS" JOURNAL OF MOLECULAR EVOLUTION, SPRINGER VERLAG, NEW YORK, NY, US, Bd. 2, Nr. 3, Juni 1995 (1995-06), Seiten 128-144, XP000892117 ISSN: 0022-2844
• KIRSTEIN F ET AL: "A molecular marker for the identification of the zoonotic reservoirs of Lyme borreliosis by analysis of the blood meal in its European vector Ixodes ricinus." APPLIED AND ENVIRONMENTAL MICROBIOLOGY, Bd. 62, Nr. 11, 1996, Seiten 4060-4065, XP002238491 ISSN: 0099-2240
• ZEHNER R ET AL: "RFLP and sequence analysis of the cytochrome b gene of selected animals and man: Methodology and forensic application." INTERNATIONAL JOURNAL OF LEGAL MEDICINE, Bd. 111, Nr. 6, Oktober 1998 (1998-10), Seiten 323-327, XP002234796 ISSN: 0937-9827
• KOCHER T D ET AL: "DYNAMICS OF MITOCHONDRIAL DNA EVOLUTION IN ANIMALS AMPLIFICATION AND SEQUENCING WITH CONSERVED PRIMERS" PROCEEDINGS OF THE NATIONAL ACADEMY OF SCIENCES OF USA, NATIONAL ACADEMY OF SCIENCE. WASHINGTON, US, Bd. 86, Nr. 16, 1989, Seiten 6196-6200, XP002189444 ISSN: 0027-8424

**Beschreibung**

GEBIET DER ERFINDUNG

Diese Erfindung betrifft ein Verfahren und einen Kit zur Bestimmung der Anwesenheit oder Abwesenheit von tierischem Zellmaterial spezifischer Tierarten oder Klassen von Tierarten in Proben, die tierisches Material enthalten, durch DNA Analyse.

HINTERGRUND DER ERFINDUNG

Der Nachweis der Anwesenheit oder Abwesenheit von Material unterschiedlicher Tierarten in Fleischprodukten, Fertiggerichten, in anderen prozessierten Lebensmitteln oder in Tierfutter ist ein wichtiger Teil der Qualitätssicherung, da häufig tierisches Material in Produkten enthalten ist, das nicht oder falsch deklariert wird.

Es ist bereits ein Verfahren zur Identifizierung von Material spezifischer Tierarten in Lebensmitteln bekannt (R. Meyer et al. (1995) Journal of AOAC International, Vol. 78, 1542-1551), das in Form eines Kits vertrieben wird. Bei diesem Verfahren wird ein Fragment des mitochondrialen Cytochrom b Gens mit Hilfe von Primern unter Anwendung des PCR-Verfahrens amplifiziert. Das PCR-Produkt wird dann mittels Restriktionsfragment-Längenpolymorphismus-Analyse einer Tierart zugeordnet. Dieses Verfahren weist jedoch eine Anzahl schwerwiegender Nachteile auf, wodurch es unter anderem für den Durchsatz einer Vielzahl von Proben ungeeignet ist. Es erfordert Restriktionsenzyme, deren Qualität und Aktivität Schwankungen unterworfen ist und die teuer sind. Ferner muss jeder Restriktionsverdau der Agarose-Gelelektrophorese unterworfen werden, was zeigt- und arbeitsaufwendig und kaum automatisierbar ist: Ferner führt das Verfahren zu einem hohen Anteil an falsch positiven bzw. falsch negativen Ergebnissen oder zu nicht eindeutigen Ergebnissen.

US 6,015,664 beschreibt einen Multiplex-PCR-Assay unter Verwendung ungleicher Primerkonzentrationen zur Detektion von HPIV 1,2,3 und RSV A,B sowie Influenza Virus A, B.

Biotechniques 12 (1992) 408-411 beschreibt ein Verfahren zur Identifikation der tierischen Quelle von biologischen Proben durch Amplifikation eines Teils des Cytochrom b Gens mittels PCR und Sequenzieren des PCR-Produkts.

DE 196 29 166 beschreibt ein analytisches Verfahren zur Festellung der Abstammung verschiedener Materialien biologischer Herkunft, wobei ein Teil des Cytochrom b Gens mittels PCR und fluoreszenzmarkierten Primern amplifiziert wird, gefolgt von einer RFLP-Analyse.

Journal of Molecular Evolution 2(1995) 128-144 beschreibt die Evolution des Cytochrom b Gens von Säugetieren

Proc. Natl. Acad. Sci. USA 86 (1989) 6196-6200 beschreibt die Dynamik der Evolution mitochondrialer DNA in Säugetieren.

Appl. Environ. Microbiol. 62 (1996) 4060-4065 beschreibt molelulare Marker zur Identifizierung des zoonotischen Reservoirs der Lyme-Borreliose durch Analyse der Blutaufnahme des Vektors *Ixodes ricinus,* wobei degenerierte PCR-Primer zur Amplifizierung orthologer Regionen des Cytochrom b Gens eingesetzt werden.

Es ist daher Aufgabe dieser Erfindung, ein Verfahren zur Bestimmung der Anwesenheit oder Abwesenheit von tierspezifischer DNA in Proben bereitzustellen, das weder Restriktionsenzyme noch Gelelektrophorese benötigt, für einen hohen Probendurchsatz geeignet ist und einen geringen Anteil falscher Ergebnisse lierfert.

Ferner ist es Aufgabe der Erfindung, ein Verfahren der genannten Art zu schaffen, das eine Vielzahl von Proben parallel zu analysieren gestattet.

Weiterhin ist es Aufgabe der Erfindung, ein Verfahren der genannten Art zu schaffen, das mit einfachen Mitteln die Anwesenheit oder Abwesenheit von tierischem Zellmaterial einer Vielzahl von Tierarten parallel zu analysieren gestattet.

ALLGEMEINE BESCHREIBUNG DER ERFINDUNG

Die Erfindung stellt ein Verfahren zur Bestimmung der Anwesenheit oder Abwesenheit von tierartspezifischer DNA in einer Probe zur Verfügung, wobei das Verfahren folgende Schritte umfasst:

(a) Bereitstellen von für die Zwecke einer Polymerase-Kettenreaktion (PCR) geeigneter DNA aus der Probe,
(b) Durchführen einer PCR Reaktion mit der DNA von Schritt (a) unter Verwendung eines markierte Primer umfassenden Primergemischs zur Herstellung eines PCR-Produkts, wobei die Primer mit konservierten Bereichen eines in den zu bestimmenden Tieren ubiquitären Gens hybridisieren und wobei das PCR-Produkt einen über alle interessierenden Tierarten oder Tierrassen variablen Bereich mit einer tierartspezifischen Sequenz aufweist;
(c) Immobilisierung dies markierte DNA-Sequenzen enthaltenden PCR-Produkts aus Schritt (b)
(d) Herstellung von immobilisierter Einzelstrang-DNA des immobilisierten PCR-Produkts aus Schritt (c),

(e) Behandlung der immobilisierten Einzelstrang-DNA aus Schritt (d) unter Hybridisierungsbedingungen mit mindestens einer tierartspezifischen Sonde,

(f) Detektion einer Hybridisierung in Schritt (e) zur Bestimmung der Anwesenheit oder Abwesenheit der tierspezifischen DNA,

wobei das ubiquitäre Gen für mitochondriales Cytochrom b codiert.

Weiterhin wird ein System zur Durchführung des obigen Verfahrens bereitgestellt. Bevorzugte Ausführungsformen werden in den Unteransprüchen beschrieben.

Gemäß dem Verfahren dieser Erfindung wird aus einer Lebensmittel- oder Tierfutterprobe eine DNA Präparation erstellt und diese DNA auf ihre tierische Herkunft untersucht. Hierzu wird ein Abschnitt eines ubiquitären Gens oder einer ubiquitären DNA Sequenz mittels der Polymerase-Kettenreaktion (PCR) amplifiziert, wobei der DNA Abschnitt einen Bereich mit einer solchen Variabilität umfasst, dass Tierarten anhand der Sequenz voneinander unterschieden werden können. Dieser variable Bereich soll von Bereichen flankiert sein, die im Rahmen der relevanten Tierarten hochkonserviert sind, so dass der DNA-Abschnitt mit Hilfe von Primern, die mit den hochkonservierten Bereichen hybridisieren, unabhängig von der Herkunft der DNA amplifiziert werden kann.

Prinzipiell eignen sich viele ubiquitäre Gene oder DNA Sequenzen für dieses Verfahren, solange die Abfolge von einem variablen Bereich und flankierenden hochkonservierten Bereichen vorhanden ist. Ersterer soll anhand seiner Sequenz eine eindeutige Zuordnung zu jeder speziellenTierart aus einer Klasse der relevanten Tierarten erlauben. Vorzugsweise ist der Abstand der konservierten Bereiche so gewählt, dass das amplifizierte PCR-Produkt für das PCR-Verfahren eine geeignete Länge hat. Das ubiquitäre Gen ist vorzugsweise mehrfach in einer Zelle vorhanden, was die Nachweisempfindlichkeit erhöht. Insbesondere weisen mitochondriale Gene diese Eigenschaft auf. Es hat sich überraschenderweise anhand eines Alignments einer Vielzahl von mit hoher Genauigkeit sequenzierter mitochondrialer Gene des Cytochrom b verschiedener Tierarten gezeigt, dass es einen ausgezeichneten Sequenzbereich gibt mit den folgenden Merkmalen:

a) Der ausgezeichnete Sequenzbereich umfasst eine Abfolge der folgenden Unterbereiche: ein erster konservierter Bereich, ein variabler Bereich, ein zweiter konservierter Bereich. Diese müssen nicht unmittelbar aneinander grenzen, solange das PCR-Verfahren bei den verwendeten Proben erfolgreich durchführbar ist.

b) Der variable Bereich ändert seine Sequenz von Tierart zu Tierart in ausreichendem Maße für eine Diskriminierung mit DNA Sonden.

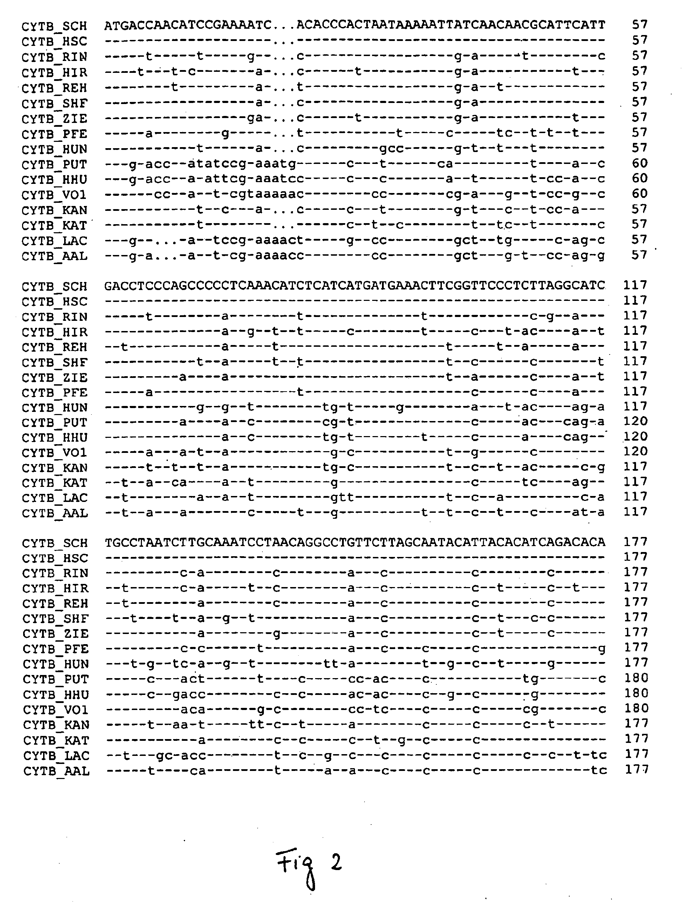

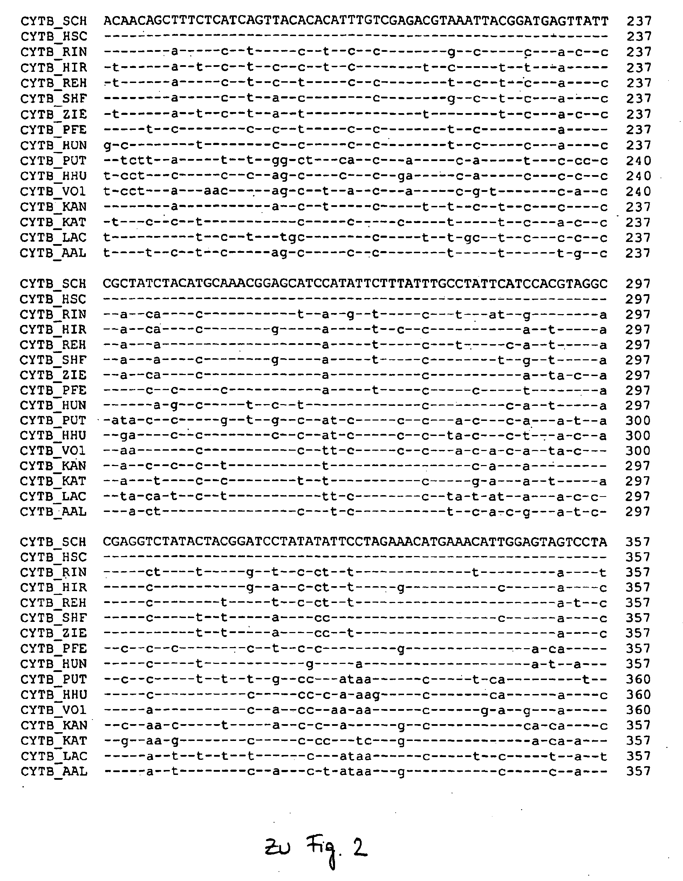

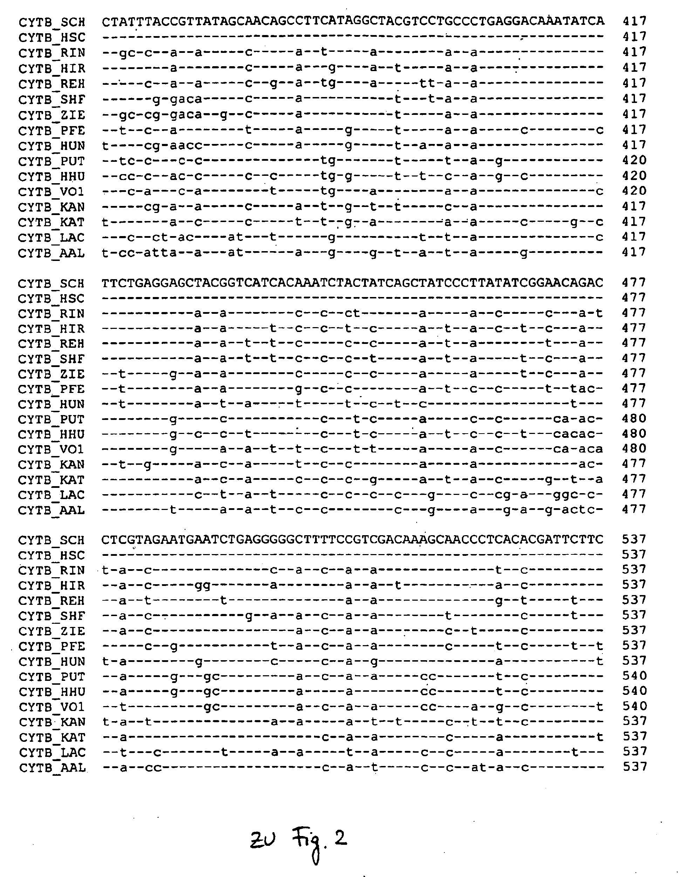

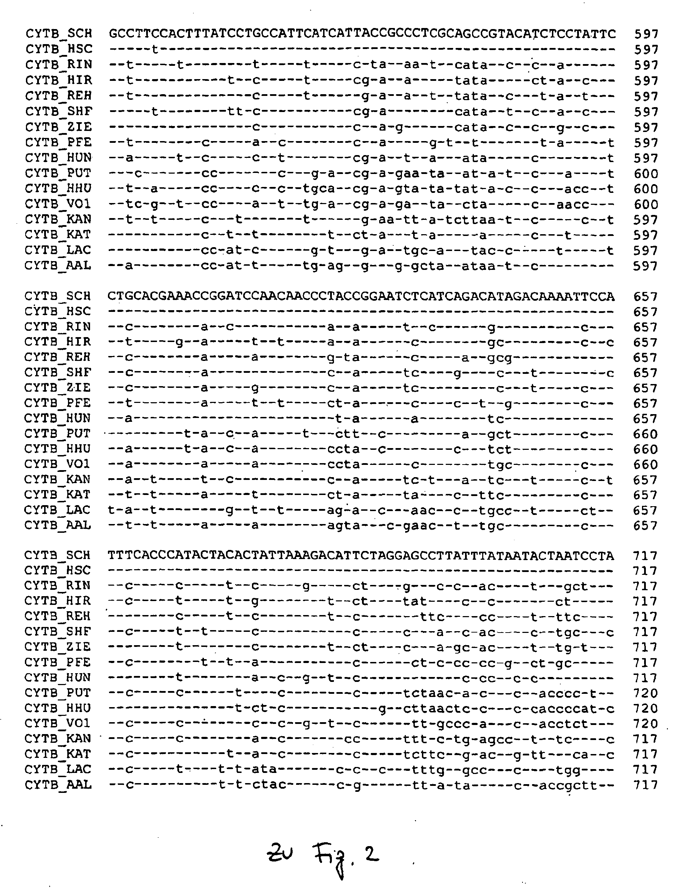

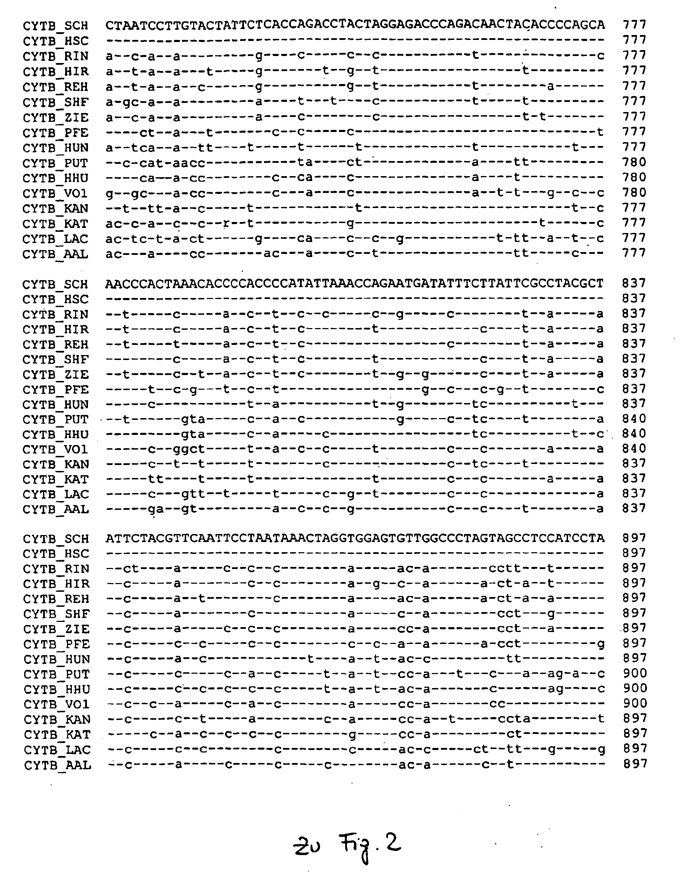

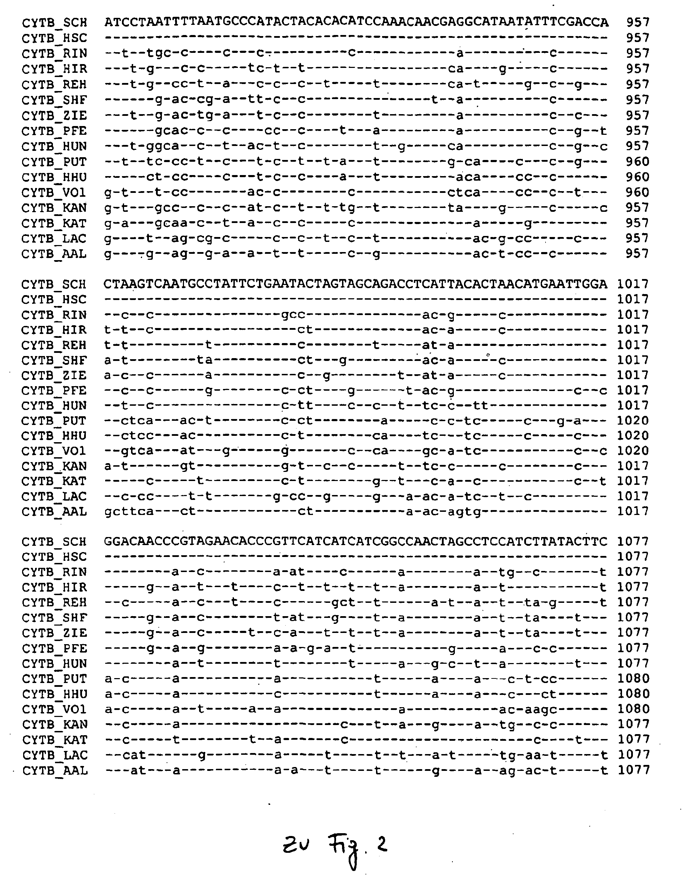

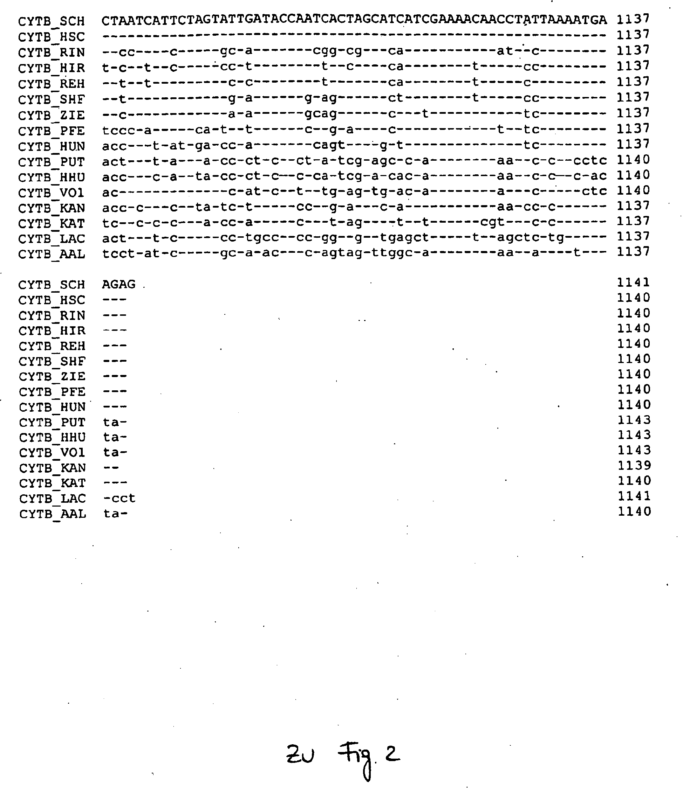

c) Der variable Bereich reicht stromauf vorzugsweise bis zur Position 666 bezogen auf das Cytochrom b Gen des Schweins als Referenzsequenz gemäß Figur 2 und stromab bis zur Position 750. Er kann weiter oder enger sein, solange die anschließenden konservierten Bereiche mit Primern für das PCR-Verfahren hinreichend hybridisierbar sind und solange die gewünschte Diskriminierung möglich ist.

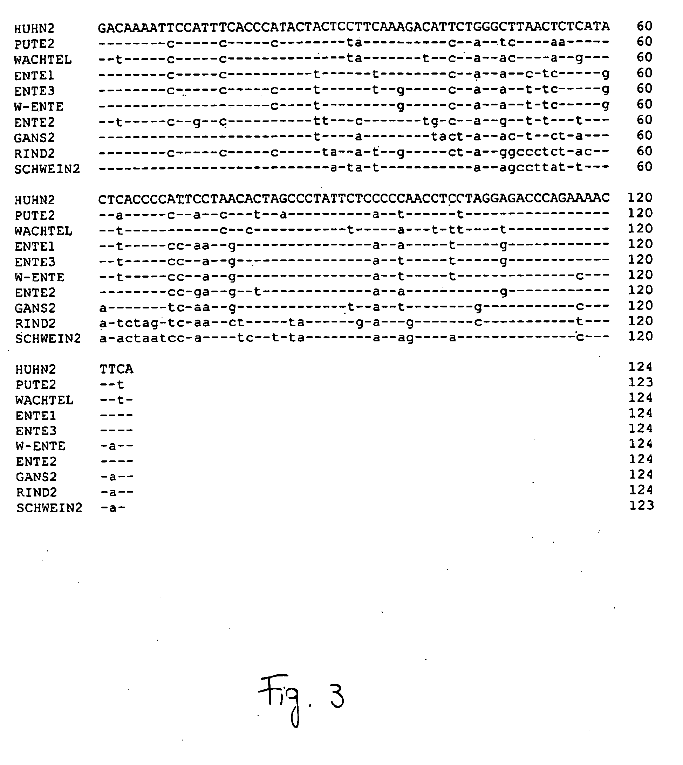

d) Der stromauf gelegene konservierte Bereich reicht vorzugsweise von Position 646 bis Position 665. Der stromab gelegene konservierte Bereich reicht vorzugsweise von Position 751 bis Position 769 der Referenzsequenz. Weitere Sequenzen sind in Figur 3 im Alignment gezeigt.

e) Der variable Bereich ist mit Sonden für alle in Frage kommenden Tierarten spezifisch hybridisierbar. Die konservierten Bereiche sind hinreichend konserviert, so dass mit einem Primergemisch gearbeitet werden kann, welches nur eine kleine Anzahl von Primern (z.B. 1 bis 5, vorzugsweise 1 bis 4 und speziell 1 bis 3) für jede Richtung erfordert.

Es kommen alle Tierarten in Betracht, die als Zellen oder als Zellfragmente in Lebensmitteln oder Futtermitteln in signifikanter Menge vorkommen können. Das Verfahren eignet sich zur parallelen diskriminierenden Erfassung mehrerer verschiedener Tierarten in einer oder mehreren Proben, oder aber zur diskriminierenden Erfassung einer speziellen, gegebenenfalls mehrere Rassen umfassenden Tierart, z.B. Rind oder Schwein.

Das PCR-Produkt sollte einerseits möglichst klein sein, da besonders in prozessierten Lebens- oder Tierfuttermitteln die DNA teilweise fragmentiert vorliegt, was die Amplifizierung erschwert. Andererseits sollte es groß genug sein, damit anhand des variablen Bereiches eine eindeutige Zuordnung zu einer Tierart getroffen werden kann. Diese Länge des PCR-Produktes beträgt vorzugsweise weniger als 200 Basenpaare (bp), besser weniger als 150 bp und am besten ≤125bp.

Beim Verfahren dieser Erfindung wird das PCR-Produkt vorzugsweise auf der Oberfläche eines Trägers immobilisiert. Zu diesem Zweck weist mindestens einer der für die PCR benutzten Primer eine Markierung auf, die die Immobilisierung erlaubt. Methoden zur Immobilisierung von DNA sind bereits bekannt. Sie können z.B. auf dem Prinzip der molekularen Erkennung beruhen. Die Markierung der Primer kann ein Molekül sein, das von einem immobilisierten Protein mit hoher Affinität gebunden wird, wie z.B. ein Antigen, das von einem immobilisierten Antikörper gebunden wird. In einer bevorzugten Ausführungsform dieser Erfindung ist die Markierung eine Biotinylierung, die von immobilisiertem Streptavidin erkannt und gebunden wird.

Das benutzte Primergemisch enthält mindestens zwei Primer, einen Vorwärts- und einen Rückwärtsprimer.

Mindestens einer dieser Primer muss zumindest teilweise eine Markierung aufweisen. Es können jedoch auch komplexere Primergemische mit mehr als einem Vorwärts- oder Rückwärtsprimer eingesetzt werden, wobei ein oder mehrere Primer vollständig oder teilweise markiert sein können. Dies kann beispielsweise dann vorteilhaft sein, wenn der Grad der Konservierung in den konservierten Bereichen nicht ausreicht, um mit nur einem Primerpaar den fraglichen Genabschnitt aller in Frage kommender Tierarten zu amplifizieren. In diesem Fall sind vorzugsweise zumindest alle Primer für eine Richtung markiert. Ferner kann mehr als nur eine Markierung benutzt werden, was die Variabilität des Verfahrens erhöhen kann. Es ist jedoch besonders bevorzugt, nur eine Markierungsart zu benutzen und ein definiertes, für alle in Frage kommenden Tierarten benutzbares, möglichst einfaches Primergemisch zu verwenden.

Das oder die PCR-Produkte können beispielsweise auf Beads, welche magnetisch sein können, oder auf Flächen- oder Volumenelementen einer Trägeroberfläche immobilisiert werden. Die Verwendung einer Trägeroberfläche ist bevorzugt. Dies erlaubt eine Kompartimentierung der nachfolgenden Schritte, so dass idealerweise viele Bestimmungen auf engem Raum parallel durchgeführt werden können und ein Vermischen von flüssigen Reagenzien, die für verschiedene Flächen- oder Volumenelemente bestimmt sind, vermieden wird. Die Kompartimentierung einer Trägeroberfläche in Flächenelemente kann auf verschiedene Arten erreicht werden. Beispielsweise kann sie durch alternierende Oberflächenbeschaffenheit erreicht werden und/oder dadurch, dass relativ zur Größe der Elemente sehr geringe Flüssigkeitsvolumina verwendet werden. Ein Träger für Flächenelemente ist vorzugsweise ein Chip oder Mikrochip.

Eine Kompartimentierung einer Trägeroberfläche in Volumenelemente kann durch Vertiefungen in einem Träger erreicht werden. Solche Volumenelemente sind vorzugsweise die Vertiefungen einer Titerplatte.

Wenn die Oberfläche eines Trägers zum Binden der Markierung der markierten Primer verwendet wird, wird die Oberfläche nach allgemein bekannten Verfahren mit dem die Markierung bindenden Molekül, z.B. einem Protein, beschichtet. Im Falle von Volumenelementen genügt es im allgemeinen, wenn nur der Boden der Vertiefungen beschichtet ist. Im Fall von Flächenelementen können entweder nur die Flächenelemente selber, d.h. ohne eventuelle Grenzbereiche zwischen den Elementen, oder die gesamte Fläche beschichtet sein. Im letzteren Fall kann die Kompartimentierung erst durch punktuelles Auftragen der PCR-Produkte oder sogar erst durch punktuelles Auftragen der Sonden bei flächiger Immobilisierung eines PCR-Produktes entstehen. Dieser letzte Fall betrifft vor allem eine Untersuchung einer Probe mit verschiedenen tierspezifischen Sonden.

Nach dem Immobilisieren eines PCR-Produktes liegt das PCR-Produkt im allgemeinen doppelsträngig vor. Der lediglich durch Basenpaarung mit dem markierten Strang gebundene Strang wird nach allgemein bekannten Methoden entfernt. Dies kann z.B. durch Wärmebehandlung, durch Zugabe von schmelzpunkterniedrigenden Substanzen, durch Denaturierung oder mittels eines kombinierten Verfahrens geschehen. Vorzugsweise wird der zu entfernende Strang durch Waschen der Trägeroberfläche endgültig beseitigt.

In Schritt (e) des Verfahrens dieser Erfindung wird die immobilisierte Einzelstrang-DNA unter Hybridisierungsbedingungen mit mindestens einer tierartspezifischen Sonde behandelt. Hybridisierungsbedingungen sind solche Bedingungen, unter denen hinreichend komplementäre DNA Sequenzen hybridisieren. Hierzu wird die Einzelstrang-DNA mit der Sonde in geeigneter Lösung bei einer geeigneten Temperatur behandelt. Vorzugsweise wird die Hybridisierung und/oder der (die) nachfolgende(n) Waschschritt(e) zur Entfernung nichtgebundener Sonden jedoch in Gegenwart eines die Selektivität der Hybridisierung erhöhenden Salzes durchgeführt. Ein solches Salz kann ein quartäres Ammoniumsalz z. B. ein Tetraalkylammoniumsalz, insbesondere ein Tetramethyl- oder Tetraethylammoniumsalz sein. Als Anion eines solchen Salzes kommt jedes nicht störende Ion, z.B. ein Halogenid in Frage. Chlorid ist besonders bevorzugt.

Eine tierartspezifische Sonde gemäß dieser Erfindung umfasst eine DNA-Sequenz, die zu mindestens einem Teil des variablen Bereichs des PCR-Produkts dieser Tierart hinreichend komplementär ist und mit diesem hybridisieren kann. Die Länge der DNA der tierartspezifischen Sonde und ihre Sequenz sollen so gewählt werden, dass die Spezifität der Hybridisierung möglichst hoch ist, d.h. dass die Sonde nur mit dem PCR-Produkt hybridisiert, das von DNA der Tierart, für die die Sonde spezifisch ist, herrührt. Kreuzreaktionen mit PCR-Produkt, das von einer anderen Tierart herrührt, sollen vermieden werden. Weitere für die Spezifität der Sonde wichtige Parameter werden wie üblich von den Hybridisierungsbedingungen (insbesondere Temperatur, Puffer, die Spezifität erhöhende Salze) gegeben.

Eine spezifische Hybridisierung der Sonde mit dem PCR-Produkt muss detektierbar sein. Wird eine Hybridisierung detektiert, gilt dies als Hinweis darauf, dass Material der Tierart, für die die Sonde spezifisch ist, in signifikanter Menge in der Probe enthalten ist. Kann kein signifikantes Signal für eine Hybridisierung detektiert werden, ist kein Material der Tierart, für die die Sonde spezifisch ist, in einer Menge, die über der Nachweisgrenze liegt, in der Probe vorhanden. Das Ergebnis kann durch Positiv- und Negativkontrollen abgesichert werden.

Es gibt eine Reihe von Möglichkeiten, eine Hybridisierung einer Sonde mit einem PCR-Produkt zu detektieren. üblicherweise wird vor der Detektion ein Waschschritt durchgeführt, um nicht hybridisierte Sonden zu entfernen. Beispielsweise kann die Sonde einen Fluoreszenzmarker enthalten, dessen Fluoreszenz messbar ist. Die Absorptions- und/oder Emmisionseigenschaften eines Fluoreszenzmarkers können auch von einer Hybridisierung abhängig sein. So markierte Sonden sind besonders für ein Verfahren auf Chip-Basis geeignet. In einem solchen Fall ist ein Waschschritt vor der Detektion entbehrlich. Ferner kann eine Sonde an ein Antigen gekoppelt sein, das von einem Antikörper erkannt und gebunden wird. Dann kann eine Hybridisierung vorteilhaft durch eine spektroskopisch, am besten absorptions- oder fluoreszenzspektroskopisch detektierbare Reaktion nachgewiesen werden, die von einem an den Antikörper gebundenen Enzym katalysiert wird. Besonders geeignet ist die Verwendung einer Peroxidase als Enzym, die eine Vielzahl von farblosen Substraten zu gefärbten Produkten oxidieren, Farbstoffe entfärben, durch Oxidation eine Farbänderungen oder Lumineszenz bewirken können. Vorzugsweise wird die Farbreaktion nach einer vorbestimmten Zeit abgestoppt, z.B. durch Zugabe einer Lösung, die die Aktivität des Enzyms stoppt.

Ein besonders nützlicher Vorteil des Verfahren zur tierartspezifischen Bestimmung von DNA dieser Erfindung liegt darin, dass es einen hohen Probendurchsatz zulässt und Schritte zur parallelen Durchführung automatisiert werden können. In einem solchen Fall ist es von Vorteil, viele Hybridisierungsreaktionen auf einem gemeinsamen Träger durchzuführen. Dies wiederum stellt besondere Anforderungen an die Spezifität der Hybridisierung der Sonde. Insbesondere wenn auf einem Träger Sonden verwendet werden, die für verschiedene Tierarten spezifisch sind, sollte die Spezifität jeder Sonde bei derselben Temperatur hinreichend gut sein. Ein wesentlicher Vorteil dieser Erfindung liegt darin, dass die Bedingungen und die Sonden, die für viele verschiedene Tierarten spezifisch sind, so aufeinander abgestimmt sind, dass Hybridisierung und Waschschritt zum Entfernen ungenügend spezifisch hybridisierter Sonden bei derselben Temperatur durchgeführt werden können. Entscheidend kann hierbei unter anderem sein, dass ein die Selektivität der Hybridisierung erhöhendes Salz zugesetzt wird. Dieses hat vorzugsweise die Wirkung, dass die Schmelzpunkte der verschiedenen Sonden mit komplementären Sequenzen einander angenähert werden.

Mit dem Verfahren dieser Erfindung kann beispielsweise eine Lebensmittel- oder Tierfutterprobe auf die Anwesenheit oder Abwesenheit von DNA von einer Vielzahl von Tierarten, vorzugsweise parallel, untersucht werden. Es können auch mehrere Proben auf die Anwesenheit von DNA einer einzigen bestimmten Tierart wie z.B. Rind untersucht werden. Durch gemeinsames Verwenden mehrerer verschiedener Sonden in einer Hybridisierungsreaktion kann auf die Gegenwart eines Vertreters aus einer beliebigen Gruppe von Tierarten getestet werden. Beispiele hierfür sind ein Test auf Geflügel durch Verwenden eines Gemischs von Sonden, die für Huhn, Pute, Ente, Gans u.s.w. spezifisch sind oder ein Test von Tierfutter auf die Anwesenheit von Tiermehl durch Verwendung eines Sondengemischs, das alle verfügbaren tierspezifischen Sonden umfasst. Diese Anwendungsmöglichkeiten können selbstverständlich auch kombiniert werden und parallel und/oder automatisiert durchgeführt werden.

Grundsätzlich kann das vorliegende Verfahren für alle Tierarten angewandt werden. Es ist jedoch besonders gut für Säugetiere, insbesondere Rind und Schwein, Fische und Vögel geeignet.

Mit dem Verfahren dieser Erfindung können selbst wenige Kopien einer tierischen DNA in einer Probe nachgewiesen werden. Eine Nachweisgrenze von 0,5% und besser für Material einer bestimmten Tierart, auf die getestet wird, kann in nahezu allen Lebensmitteln und Futtermitteln mit beliebigem Anteil an anderem tierischen Material identifiziert werden.

Das Verfahren dieser Erfindung kann auch auf Tierarten angewandt werden, deren Cytochrom b Sequenz hier nicht spezifisch beschrieben wird oder deren Cytochrom b Gen noch nicht sequenziert wurde. In diesen Genen kann die Region, die gemäß dem Verfahren dieser Erfindung amplifiziert wird, durch Sequenzalignment z.B. mit der Schweinesequenz unter Verwendung handelsüblicher Alignment-Programme und Standardeinstellungen gefunden werden.

KURZE BESCHREIBUNG DER FIGUREN

Fig. 1     zeigt schematisch eine Abfolge von Verfahrensschritten, die zur Durchführung des Verfahrens dieser Erfindung dienen.

Fig. 2     zeigt ein Alignment der Sequenzen des mitochondrialen Cytochrom b Gens von Schwein (SCH), Hausschwein (HSC), Rind (RIN), Hirsch (HIR), Reh (REH), Schaf (SHF), Ziege (ZIE), Pferd (PFE), Hund (HUN), Pute (PUT), Huhn (HHU), Vireo (Singvogel, VO1), Kaninchen (KAN), Katze (KAT), Lachs (LAC), Aal (AAL).

Fig. 3     zeigt ein Alignment des gemäß dieser Erfindung amplifizierten Bereichs des Cytochrom b Gens. Hier dient die Huhnsequenz als Referenzsequenz. Die übrigen Sequenzen enthalten nur für die Basen einen Eintrag, die sich von denen der Huhnsequenz unterscheiden.

DETAILLIERTE BESCHREIBUNG DIESER ERFINDUNG

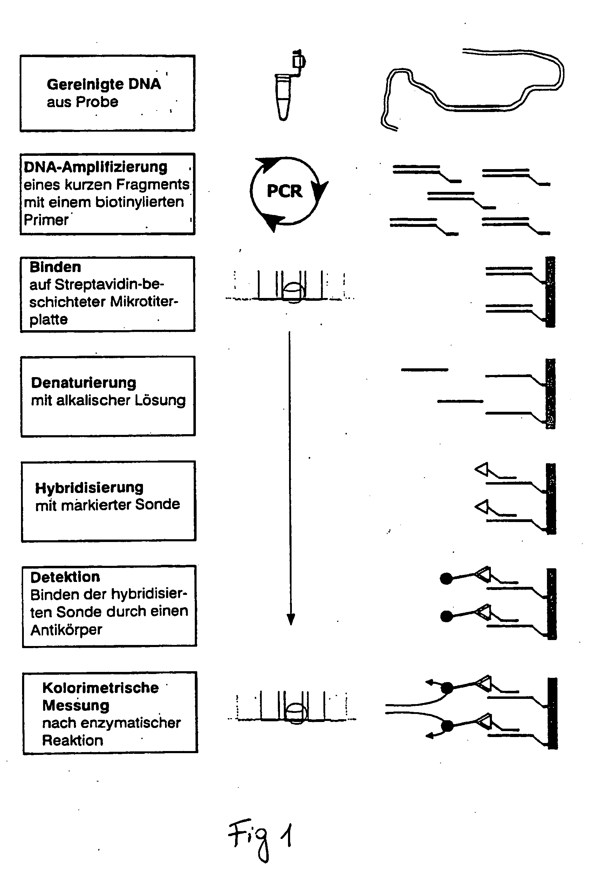

Im Folgenden werden bevorzugte Ausführungsformen dieser Erfindung beispielhaft beschrieben. Vergleiche dazu das Flussdiagramm von Figur 1.

Das Bereitstellen von für die Zwecke einer PCR geeigneter DNA aus einer Probe kann gemäß allgemein bekannten Methoden erfolgen. Üblicherweise umfassen diese Methoden eine Homogenisierung, einen Proteinaseverdau sowie eine DNA Extraktion.

Die so erhaltene DNA wird zur Amplifikation eines geeigneten Bereichs eines ubiquitären Gens, vorzugsweise des mitochondrialen Cytochrom b Gens verwendet. Als Vorwärtsprimer kann vorzugsweise Primer Cytb_CON1 (Nr.

186) verwendet werden. Als Rückwärtsprimer eignen sich z.B. die Primer Bio-Cytb_CON2 (Nr. 192) oder Bio-Cytb_CON3 (Nr. 227). Diese Primer haben die folgenden Sequenzen:

Cytb_CON1 (Nr. 186), mitochondriales Cytochrom b Gen, Position 646-665:

5'-gac aaa att cca ttY cac cc-3', wobei Y für c oder t steht,

Bio-Cytb_CON2 (Nr. 192), mitochondriales Cytochrom b Gen, Position 751-769:

5'-bio-tgt agt tgt ctg ggt ctc c-3',

Bio-Cytb_CON3 (Nr. 227), mitochondriales Cytochrom b Gen, Position 751-769:

5'-bio-tgt agt tgt ctg ggt ccc c-3',

wobei bio für eine Biotinylierung der Primer steht.

Die Positionsangaben beziehen sich auf die in Figur 2 gezeigte Schweinesequenz als Referenzsequenz. Markierte Primer, insbesondere auch biotinylierte Primer sind kommerziell allgemein erhältlich. Die Verfahren zur Markierung von Oligonukleotiden mit einer Vielzahl von Markern sind bekannt.

Es hat sich als besonders vorteilhaft erwiesen, ein Gemisch der Primer Bio-Cytb_CON3 und Bio-Cytb_CON2 zu verwenden, insbesondere, wenn auch auf Ente und Wachtel untersucht wird.

Die PCR erfordert keine speziellen Bedingungen. Üblicherweise werden Taq Polymerase und 30-40 Zyklen verwendet. Es sollte jedoch für die PCR eine Positivkontrolle und eine Negativkontrolle ohne DNA-Templat durchgeführt werden. Für die Positivkontrolle kann ein das Cytochrom b Gen enthaltendes Plasmid oder DNA aus einer Fleischprobe bekannter tierischer Herkunft benutzt werden. Desweiteren empfiehlt es sich, für jede zu untersuchende Probe zwei PCR-Reaktionen durchzuführen, wobei eine der beiden als interne Positivkontrolle DNA der zu testenden Tierart zugeben wird. Diese Kontrolle dient zur Überprüfung auf Inhibitoren, die in der Probe enthalten sein und einen der Schritte des Verfahrens inhibieren können.

Die Immobilisierung des PCR-Produktes erfolgt im Fall von biotinmarkierten Primern über Streptavidin. (Mikro-) titerplatten mit Streptavidin-beschichteten Vertiefungen sind kommerziell erhältlich. Das darauf befindliche Streptavidin wird vor der Immobilisierung mit dem Rehydratisierungspuffer (siehe unten) hydratisiert.

Das PCR-Produkt wird dann üblicherweise mit dem Bindungspuffer (siehe unten) verdünnt, und zur Immobilisierung in eine oder mehrere Vertiefungen der Streptavidin-beschichteten Titerplatte gegeben. Die Immobilisierung erfolgt vorzugsweise bei erhöhter Temperatur, z.B. um 42°C.

Anschließend wird von dem immobilisierten PCR-Produkt Einzelstrang-DNA durch Entfernen der durch Hybridisierung an die markierten DNA-Stränge gebundenen Stränge hergestellt. Dies kann beispielsweise mit einer Denaturierungslösung wie 0,25 N NaOH gefolgt von Waschen erreicht werden.

Die Hybridisierungssonden werden in Hybridisierungspuffer (siehe unten) verdünnt und bei erhöhter Temperatur mit der immobilisierten Einzelstrang-DNA inkubiert. Die Inkubationstemperatur beträgt bei den unten genannten Sonden zwischen 39 und 45°C, vorzugsweise 42°C. Im folgenden werden Sequenzen (in 5'-3'-Richtung) der Sonden für eine Reihe von Tierarten gegeben, die für das Verfahren dieser Erfindung besonders geeignet sind. Diese Sonden verfügen über eine hervorragende Spezifität für die jeweilige Tierart. Insbesondere führen diese Sonden bei den angegebenen Bedingungen zu keinen nennenswerten Kreuzreaktionen mit PCR-Produkt einer anderen Tierart.

Putensonde: FITC-aat cat act aac ccc ctt a

Hirschsonde: FITC-atc tta ctt cta gta ctc ttc

Schweinssonde: FITC-ata cta atc cta cta atc c

Rindsonde: FITC-cct ctt act aat tct agc

Huhnsonde: FITC-tct cat act cac ccc att c

Schafsonde 1: FITC-tgc tat cct act aat cct ca

Schafsonde 2: FITC-cct cat gct act agt act

Entensonde: FITC-tgt cct agg gtt tat tct t

Ganssonde: FITC-ctt aat aat cac ccc tct a

Pekingente: FITC-tcc tca tgc tta ccc cc

Hierbei steht FITC für Fluorescinisothiocyanat, mit dem die Sonden markiert sein können.

Die obengenannten Sonden weisen die überaschende Eigenschaft auf, dass ihre Schmelzpunkte mit ihren Zielsequenzen eng beieinander liegen. Somit können verschiedene Sonden auf einem Träger bei derselben Hybridisierungstemperatur und derselben Waschtemperatur benutzt werden. Dies ist insbesondere dann der Fall, wenn ferner ein die Selektivität der Hybridisierung erhöhendes Salz bei der Hybridisierung oder dem anschließenden Waschschritt eingesetzt wird. In einer besonders geeigneten Ausführungsform wird ein Tetraalkylammoniumsalz, am besten Tetramethylammoniumchlorid in einem Waschpuffer zum Entfernen unspezifisch gebundenen Sonden verwendet. Es ist insbesondere auch die Kombination vorteilhafter Merkmale dieser Erfindung, nämlich die Sequenzen der Sonden, die Wahl des zur Bestimmung verwendeten Zielgens, die Wahl des amplifizierten Bereiches des Zielgens und damit auch die Wahl der Sequenzen der PCR-Primer, sowie das die Selektivität der Hybridisierung erhöhende Salz, die das Verfahren dieser Erfindung gegenüber den Verfahren des Standes der Technik überlegen macht.

Zur Absicherung der Ergebnisse kann beim Hybridisierungsschritt eine Positiv- und eine Negativkontrolle gemacht werden. Für die Positivkontrolle wird eine Sonde benutzt, die bei den verwendeten Bedingungen mit einer Zielsequenz hybridisieren muss. Als Negativkontrolle kann eine Sonde benutzt werde, deren Sequenz geringfügig von der in der Positivkontrolle verwendeten abweicht. Diese darf bei den verwendeten Bedingungen nicht mit einer Zielsequenz hybridisieren.

Sequenzen (in 5'-3'-Richtung) geeigneter Sonden, die als Hybridisierungskontrolle dienen können, sind:

Positivkontrolle: FITC-tag agt ccc tta cgg ata ca
Negativkontrolle: FITC-tag agt ccc gca att ata ca.

Nach der Hybridisierung sollte mindestens ein, am vorteilhaftesten zwei Waschschritte zur Entfernung unspezifisch gebundener Sonden ausgeführt werden. Beispiele für einen solchen Waschpuffer 1 und einen Waschpuffer 2 (Stringency Buffer) werden unten gegeben. Wie bereits erwähnt kann das die Selektivität der Hybridisierung erhöhende Salz in einem Waschpuffer verwendet werden. Die Konzentration eines solchen Salzes im Waschpuffer beträgt zwischen 0,5 und 3 M, besser zwischen 1 und 2,5 M und am vorteilhaftesten um 1,8 M. Das Hybridisierungsprodukt wird mit einem solchen Puffer bei erhöhter Temperatur gewaschen. Die hierfür am besten geeignete Temperatur hängt von der Sequenz der Sonden bzw. vom Zielbereich des Gens ab. Gemäß dieser Erfindung und mit den obengenannten Primern und Sonden, liegt die Waschtemperatur zwischen ca. 50 und 55°C, am besten bei 53°C.

Sind die Sonden mit einem optisch detektierbaren Marker wie einem Fluoreszenzmarker markiert, kann nun direkt auf Hybridisierung der Sonden mit dem PCR-Produkt untersucht werden. Bei Verwendung von Titerplatten eignet sich hierzu beispielsweise ein käuflicher Plate Reader, der die Fluoreszenz der Lösung in den Kavitäten der Titerplatte messen kann. Die Messung der Fluoreszenz erfolgt vorzugsweise in der Nähe des Emissionsmaximums des Markers. Im Fall von einer Fluorescinmarkierung der Sonden ist dies um 500 nm.

Soll die Hybridisierung der Sonden absorptionspektroskopisch bestimmt werden, so kann die Sonde über ihre Markierung an einen Antikörper gebunden werden, der wiederum an ein eine Farbreaktion katalysierendes Enzym gebunden ist. Zur Verhinderung unspezifischer Antikörperbindung kann nach den Waschgängen ein BSA (Rinderserumalbumin) -haltiger Blockierpuffer (siehe unten) benutzt werden. Als antigene Markierung der Sonde kann irgendein Hapten benutzt werden, gegen das Antikörper hergestellt werden können. Beispiele für solche Haptene sind organische Moleküle, die die Detektion nicht inhibieren, u.a. auch Peptide. Vorteilhaft können auch fluoreszenz- oder absorptionsspektroskopisch detektierbare organische Moleküle verwendet werden. Dann kann die Hybridisierung wahlweise fluoreszenz- bzw absorptionsspektroskopisch oder unter Verwendung der Antikörper-Enzym-Reaktion untersucht werden. In letzterem Fall handelt es sich bei dem Hapten am besten um einen Fluoreszenzmarker oder -farbstoff, wie z.B. Fluorescin. Die enzymatische Detektion hat gegenüber derjenigen mit einem Fluoreszenzmarker den Vorteil, dass das Enzym einen Verstärkungseffekt bewirkt, so das die Sensitivität im allgemeinen besser ist. Antikörper gegen Fluorescin, die an eine Peroxidase gekoppelt sind, sind im Handel erhältlich.

Nach Entfernen des Blockierpuffers wird der an das Enzym gebundene Antikörper in einem geeigneten Puffer (Konjugatpuffer, siehe unten) in die Vertiefungen gegeben und vorzugsweise bei Raumtemperatur inkubiert. Die Inkubationszeit soll mindestens 10 min betragen, 30 min ist besonders empfehlenswert. Anschließend sollten nichtgebundene Antikörper gründlich weggewaschen werden z.B. mit unten genanntem Waschpuffer 3.

Nun erfolgt die Zugabe eines oder mehrerer Substrate für das antikörpergebundene Enzym. Bei diesem handelt es sich um irgendein Enzym, das Farb- oder Lichtreaktionen katalysieren kann. Besonders geeignet ist eine Peroxidase, wie z.B. Meerettich-Peroxidase. Peroxidasen erfordern vorzugsweise mindestens zwei Substrate: Ein Peroxid, vorzugsweise ein organisches Peroxid oder Wasserstoffperoxid und ein Substrat, dessen Oxidation zu einer Farbänderung oder Lumineszenz führt. Als Substrat, dessen Oxidation zu einer Farbänderung oder Lumineszenz führt, eignen sich wegen der breiten Substratspezifität der Peroxidasen viele Verbindungen. Aromatische Amine und Phenole sind bevorzugt. Ein gängiges Substrat ist z.B. Tetramethylbenzidin (TMB). Selbstverständlich kann auch ein Substrategemisch eingesetzt werden.

Die Inkubationszeit sollte so sein, dass sich in den Positivkontrollen ein deutliches Signal entwickeln kann. 15 min sind in der Regel geeignet. Dann kann die enzymatische Reaktion durch Zugabe einer Stoplösung gestoppt werden. Eine solche inhibiert die Enzymaktivität z.B. durch einen Inhibitor oder durch Denaturieren mit Säure oder Base. Denaturieren ist bevorzugt. Beispiele für eine Stoplösung sind 5% Schwefelsäure oder 5% Salzsäure. Dabei kann es bei Substraten oder Produkten der enzymatischen Reaktion zu einem Farbumschlag kommen, wenn wie bei TMB ionisierbare Gruppen vorliegen.

Das Abstoppen nach einer definierten Zeitspanne hat den Vorteil, dass dann aus der Farbintensität zumindest teilweise quantitative Information bezüglich des Gehalts von Material der detektierten Tierart gewonnen werden kann.

Die Absorption in den Vertiefungen einer Titerplatte kann rein visuell, vorzugsweise jedoch mit einem kommerziellen Plate Reader bestimmt werden. Die Wellenlängen, bei denen gemessen wird, hängen von dem Substrat des Enzyms ab. Im Fall einer Peroxidase und TMB als Substrat kann bei 450 nm gemessen werden. 620 nm eignet sich als Referenzwellenlänge.

Die Absorptionsmessung sollte kurz nach dem Abstoppen erfolgen, da sich die Absorption der Lösung infolge von Luftoxidationsprozessen verändern kann.

Vor der Auswertung der Messung werden zunächst die Kontrollreaktionen kontrolliert. Die PCR- und die Hybridisierungspositivkontrolle müssen positiv sein. Die PCR (ohne Templat)- und die Hybridisierungsnegativkontrolle müssen negativ sein. Weiterhin wird eine Kontrolle als positiv eingestuft wird, wenn deren Absorption mindestens doppelt so groß ist, wie die der Negativkontrollen. Die Absolutwerte der Absorption, bei der eine Reaktion als positiv oder negativ eingestuft wird, hängt vom Detektionssystem und von den Chargen der verwendeten Materialien ab. Bei der Verwendung von Peroxidase/TMB ist die Absorption größenordnungsmäßig $\leq$ 0,1-0,3, häufig $\leq$ 0,15-0,2, damit eine Kontrolle als negativ eingestuft wird. Die Absorption einer Positivkontrolle muss entsprechend größer sein, im allgemeinen bei >0,15-0,25. Eine Probe wird dann als tierartpositiv bewertet, wenn die Absorptionswerte für das Nachweisgen und die interne Positivkontrolle im obengenannten Bereich liegen, also >0,15-0,25, und mindestens doppelt so groß sind wie die entsprechenden Negativkontrollen.

Ist das Ergebnis der Proben-DNA negativ, muß der Absorptionswert der internen Positivkontrolle (Proben-DNA + positive control DNA) mindestens 50 % des Absorptionswertes der positive control PCR betragen. Andernfalls sind Inhibitoren in der Probe vorhanden, welche die PCR-Reaktion unterdrücken. (Siehe unten stehendes Beispiel). Unter diesen Bedingungen kann keine Aussage getroffen werden, da ein falsch negatives Ergebnis möglich ist. Isolierung und Reinigung der DNA müssen verbessert werden.

Puffer und Lösungen, die für diese Erfindung verwendet werden können, insbesondere für die Hybridisierung einer Mikrotiterplatte:

Alle Reagenzien haben p.a. Reinheit.

**Rehydratisierungspuffer (10x PBS Puffer, Herstellung von 1l):**

| | |
|---|---|
| 0,58 M $Na_2HPO_4 \cdot 2H_2O$ | 103,2 g |
| 0,17 M $NaH_2PO_4 \cdot H_2O$ | 23,5 g |
| 0,68 M NaCl | 40 g |

mit Millipore Milli-Q Wasser auf 900 ml auffüllen und pH auf 7,4 einstellen, auf 1ℓ auffüllen, autoklavieren. Für den Einsatz auf 1 x Puffer verdünnen.

**10x PBS Puffer (Herstellung von 1l):**

| | |
|---|---|
| 26,8 inM KCl | 2 g |
| 17,6 mM $KH_2PO_4$ | 2,4 g |

(fortgesetzt)

| 1,36 M NaCl | 80 g |
| 100 mM $Na_2HPO_4 \cdot 2H_2O$ | 17,8 g |

mit Millipore Milli-Q Wasser auf 900 mℓ auffüllen und mit HCL pH auf 7,4 einstellen 1 l auffüllen, autoklavieren.

**1 M Tris-HCl pH 8.0 (200 ml):**

1 M Tris    24,2 g

mit Millipore Milli-Q Wasser auf 180 ml auffüllen pH 8,0 mit HCl konz. einstellen, auf 200 mℓ auffüllen, Lösung autoklavieren.

**0.5 M EDTA pH 8.0 (200 ml):**

0,5 M EDTA·$2H_2O$    37,2 g

auf 180 ml mit Millipore Milli-Q Wasser auffüllen und pH 8,0 mit 10% NaOH einstellen. Lösung auf 200 ml mit Millipore Milli-Q Wasser auffüllen. Autoklavieren.

**200 mM Phosphatpuffer pH 7,7 (Herstellung von 200 ml):**

Folgende Lösungen einzeln herstellen (je 200 ml):

| Lösung A: 200 mM $Na_2HPO_4 \cdot 2H_2O$ | 7,12 g |
| Lösung B: 200 mM $NaH_2PO_4.H_2O$ | 5,52 g |

Lösung A vorlegen und Lösung B zugeben bis pH 7,7 eingestellt ist. Anschließend Lösungen autoklavieren

**50 x Denhardt's Reagenz (Herstellung von 50 ml):**

| 1 % BSA (Rinderserumalbumin) Fraktion V | 0,5 g |
| 2 % Polyvinylpyrrolidone | 0,5 g |
| 1% Ficoll Typ 400 | 0,5 g |

in 50 ml Millipore Milli-Q Wasser lösen und über 0,2 μm Filter steril filtrieren. Lagerung von Gebrauchsmengen bei 4°C. Vorratsmengen bei -20°C lagern.

**Bindungspuffer (Zum Verdünnen der PCR Produkt, Herstellung von 200 ml):**

| 10 mM Tris-HCl pH 8,0 | 2 ml einer 1 M Lösung |
| 0,5 M NaCl | 5,84 g |
| 1 mM EDTA | 200 μl einer 0,5 M Lösung |

auf 200 ml mit Millipore Milli-Q Wasser auffüllen, anschließend Lösung autoklavieren

**Denaturierungslösung (Herstelung von 100 ml):**

<div align="center">0,25 N NaOH     1 g</div>

auf 100 ml mit Millipore Milli-Q Wasser auffüllen.

**Hybridisierungspuffer (Herstellung von 10 ml):**

| | |
|---|---|
| 0,9 M NaCl | 1,8 ml einer 5 M NaCl Lösung |
| 50 mM NaP04 (pl17,7) | 2,5 ml des 200 mM Phosphatpuffers pH 7,7 |
| 5 mM EDTA | 100 $\mu$l einer 0,5 M EDTA Lösung pH 8,0 |
| 10% 50x Denhardt's Reagenz | 1 ml von 50x Denhardt's Reagenz |

mit 4,6 ml Millipore Milli-Q Wasser auffüllen. Alle Lösungen werden bei der Herstellung autoklaviert. Nach dem Mischen wird der Hybridisierungspuffer steril filtriert. Bei 4°C lagern.

**Waschpuffer 1 (Herstellung von 200 ml):**

| | |
|---|---|
| 0,9 M NaCl | 10,5 g |
| 50 mM NaP04 (pH 7,7) | 50 m$\ell$ des 200 mM Phosphatpuffers pH 7,7 |
| 5 mM EDTA | 2 ml einer 0,5 M EDTA Lösung pH 8,0 |

auf 200 ml mit Millipore Milli-Q Wasser auffüllen und autoklavieren. Lagerung bei Raumtemperatur.

**Waschpuffer 2 (Stringency Buffer, Herstellung von 10 ml):**

Benötigt werden 150 $\mu$l pro Vertiefung. Die Herstellung erfolgt aus autoklavierten Lösungen. Lösungen steril zusammen pipettieren.

| | |
|---|---|
| 3 M Tetramethylammoniumchlorid TMAC | 6 ml einer 5 M Lösung |
| 50 mM Tris-HCl pH 8,0 | 500 $\mu$l einer 1 M Tris pH 8,0 Lösung |
| 2 mM EDTA | 40 $\mu$l einer 0,5 M EDTA Lösung pH 8,0 |
| 0,025 Triton X-100 | 2,5 $\mu$l Triton X-100 |

mit 3458 $\mu$l Millipore Milli-Q Wasser auffüllen. Lagerung bei Raumtemperatur.

**Blockierpuffer nach CONGEN (Herstellung von 100 m$\ell$)**

| | |
|---|---|
| 3 % BSA Fraktion V | 3 g |
| 0,1 % Tween 20 | 100 $\mu$l |

in 1 x PBS (pH 7,4)

Sterilfiltrieren über 0,2 $\mu$m Filter. Lagerung bei 4°C oder portionsweise einfrieren.

Puffer für die Detektion:

**Konjugatpuffer (Herstellung von 100 ml)**

| | |
|---|---|
| 0,1 %Tween 20 | 100 $\mu$l |
| 1 % BSA Fraktion V | 1 g |

in 1 x PBS nach Invitrogen (pH 7,4)
Sterilfiltrieren über 0,2 $\mu$m Filter. Lagerung bei 4°C.

**Waschpuffer 3 (Herstellung von 100 ml)**

| | |
|---|---|
| 0,1 %Tween 20 | 100 $\mu$l |

in 1 x PBS nach Invitrogen (pH 7,4)
Sterilfiltrieren über 0,2 $\mu$m Filter. Lagerung bei Raumtemperatur oder 4°C.

**Antikörper**

Anti-Fluorescin Antikörper sind im Handel erhältlich (z.B. von SIGMA). Ebenso solche, die an Meerrettich-Peroxidase gekoppelt sind. Käufliche Lösungen können nach Verdünnen wie in den Beispielen angegeben verwendet werden.

**TMB Substratlösung**

15 ml Citratpuffer, 750 $\mu$l TMB Konzentrat, 4,95 $\mu$l $H_2O_2$ (30%).
Zusammensetzung des TMB Konzentrats: 50,4 mg TMB in 1 ml Aceton lösen und auf 10 ml mit Methanol auffüllen (21 mM TMB).
Citratpuffer: 210 mM Citrat, pH 3,95).
**Hybridisierungssonden werden** in 1h UV bestrahltem Wasser verdünnt auf eine Konzentration von 0,5 pmol/$\mu$l und gemäß den Beispielen verwendet.
**Herstellung des 10x PCR Puffers** (von 100 ml)
25 ml 2M KCl-Lösung (Endkonzentration 500 mM)
10 ml Tris pH 8,3 (Endkonzentration 100 mM)
65 ml Millipore-Wasser, nicht autoklaviert.
Über 0,2 $\mu$m Filter sterilfiltrieren. Dann jeweils 70 $\mu$l in Eppendor-Gefäße aliquotieren und mit offenem Deckel 30 min unter UV der Sterilbank bestrahlen. Lagerung bei 20°C.

BEISPIELE

Notwendige Ausrüstung und Materialien:

- Thermocycler (PCR-Cycler)
- Mikrotiterplattenreader 450/620 nm
- Thermomixer für Mikrotiterplatten oder Hybridisierungsofen bzw. ähnlicher Inkubator
- PCR-Reaktionsgefäße und Reaktionsgefäße für die Herstellung von Verdünnungen
- Pipettenspitzen mit Filtereinsatz (Filtertips), empfehlenswert zur Vermeidung von Kreuzkontaminationen
- Pipetten; 8-Kanal-Pipette oder 12-Kanal-Pipette bei gleichzeitiger Untersuchung mehrerer Proben empfehlenswert

**Beispiel 1: PCR**

Vor Beginn der PCR ist die Anzahl der benötigten PCR-Reaktionen für das Nachweisgen zu bestimmen. Die Anzahl der Reaktionsansätze wird nach der folgenden Formel berechnet:

## Anzahl Reaktionsansätze = 2 x Anzahl Proben + 2 PCR-Kontrollen

Die Proben-DNA wird für einen Ansatz zum Tierarten-Nachweis und einen Ansatz zur Überprüfung auf Inhibitoren eingesetzt. Die Überprüfung auf Inhibitoren erfolgt durch Zugabe der positive control DNA (interne Positivkontrolle für die nachzuweisende Tierart) zu der Proben-DNA.

Beispiel für 10 Proben

## 2 x 10 Proben + 2 PCR-Kontrollen = 22 Reaktionsansätze

Pro Reaktionsansatz für das Nachweisen:

9,9 $\mu$l PCR Mix mit 0,1 $\mu$l (1 unit) Taq Polymerase mischen.

Für jeden Reaktionsansatz 10 $\mu$l Taq PCR Mix in die PCR-Reaktionsgefäße pipettieren.
Zugabe von jeweils 2 $\mu$l der PCR-Kontroll-DNAs, der Proben-DNA, bzw. der Proben-DNA + Positivkontroll-DNA.
Alle Reaktionsansätze mit PCR grade H$_2$O zu insgesamt 20 $\mu$l auffüllen.
Zusammensetzung des PCR-Mix:

| | |
|---|---|
| 559 $\mu$l | 1 h UV bestrahltes Millipore Wasser |
| 260 $\mu$l | 10x PCR Puffer enthaltend 15 mM MgCl$_2$ |
| 130 $\mu$l | (5pmol/$\mu$l) Primer cytB_CON1 |
| 65 $\mu$l | (5pmol/$\mu$l) Primer cytB_CON2 |
| 65 $\mu$l | (5pmol/$\mu$l) Primer cytB_CON3 |
| 208 $\mu$l | dNTP Mix (2,5 M je dNTP) |

Beispiel für ein PCR-Programm: 1 min 95°C anfängliches Denaturieren, Zyklen: 20 s 95°C Denaturieren, 20 s 48 °C Annealing, 30 s 72°C Elongation, 3 min 72°C terminale Elongation, Kühlen 4-10°C. Zyklenzahl: 30-40 Zyklen, abhängig von der Probenart.
Nach der Durchführung der PCR ist eine Unterbrechung der Analyse möglich; Lagerung der PCR-Produkte bis zu 24 Stunden bei 4°C, über 24 Stunden bis zu mehreren Wochen bei -20°C.

**Beispiel 2: Binden der PCR-Produkte an Streptavidin-beschichtete Vertiefungen einer Mikrotiterplatte und Denaturierung**

Vor dem Beginn der Bindung der PCR-Produkte an die Mikrotiterplatte sollte man sich einen genauen Plan machen, welche Probe auf welche Tierart untersucht werden soll.
Berechnung der benötigten Kavitäten (Vertiefungen) für die Hybridisierung:

| | |
|---|---|
| je Test 2 Hybridisierungskontrollen | 2 Kavitäten |
| je Test 2 PCR-Kontrollen | 2 Kavitäten |
| je Probe und Tierart | 2 Kavitäten |

Beispiel für 2 Proben

Probe 1 wird untersucht auf Schaf, Ziege und Rind,
Probe 2 wird untersucht auf Schwein und Rind,

## 2 (Hybridisierungskontrollen) + 2 (PCR-Kontrollen) + 2 x 3 (3 Tierarten Probe 1) + 2 x 2 (2 Tierarten Probe 2) = 14 Kavitäten (insgesamt).

Zugabe von je 100 μl Rehydratisierungspuffer in die benötigten Streptavidin-beschichteten Kavitäten. 10 min zum Rehydratisieren bei Raumtemperatur ohne Schütteln inkubieren.

Pro Reaktionsansatz wird eine Vertiefung benötigt. Für die Hybridisierungskontrollen sind jeweils zwei zusätzliche Kavitäten erforderlich. Hierzu wird das folgende Oligonukleotid immobilisiert: bio-ttt ttt gta tcc gta agg gac tct a-3'.

## Anzahl Kavitäten = Anzahl PCR-Produkte + 2 Hybridisierungskontrollen.

PCR-Produkte und Hybridisierungskontrollen in Bindungspuffer verdünnen.
Die PCR-Produkte und die hyb-control werden 1:100 verdünnt. Pro Kavität werden 50 μl benötigt.

Thermomixer auf 42°C einstellen.

Rehydratisierungspuffer durch einfaches Invertieren aus den Kavitäten der Mikrotiterplatte entfernen. Mikrotiterplatte auf einer saugfähigen Unterlage (z.B. Kleenex) leicht ausklopfen. Zur Vermeidung von Kontaminationen muß darauf geachtet werden, dass die Mikrotiterplatte zügig invertiert wird.

Jeweils 50 μl der in PCR-Bindungspuffer verdünnten PCR-Produkte in die Mikrotiterplatte pipettieren. Inkubation bei 42°C im Thermomixer für 30 min bei ca. 300 rpm. Kavitäten (Vertiefungen) zur Vermeidung von Evaporation mit selbstklebender Mikrotiterplattenfolie abdecken.

Für die Dokumentation der Zuordnung der Hybridisierungsreaktionen in den Kavitäten wird ein Schema angefertigt.

Folie entfernen (Folie kann weiter verwendet werden, deshalb auf identische Positionierung achten). Puffer abpipettieren. Dann Zugabe von 50 μl Denaturierungspuffer. Inkubation bei Raumtemperatur ohne Schütteln für 10 min.

**Beispiel 3: Hybridisierung**

Abpipettieren des Denaturierungspuffers. Waschen der Kavitäten mit 50 μl Hybridisierungspuffer (Zugabe, kurz schütteln und wieder abpipettieren).

Die Hybridisierungssonden in wässriger Lösung in Hybridisierungspuffer 1:99 (1 μl + 99 μl oder entsprechend) verdünnen. Zugabe von 50 μl der verdünnten Hybridisierungssonden je Kavität.

Für jeden Ansatz wird eine Kavität der Mikrotiterplatte benötigt. Für jede weitere Probe sind die Proben-DNA und die interne Positivkontrolle (Proben-DNA + positive control DNA) mit der entsprechenden tierspezifischen Probe zu untersuchen.
Zu jeder Probe wird die Sonde der Tierart zugeordnet, auf die untersucht wird. Für die Positivkontrolle der PCR und die no-template control wird die Rindersonde verwendet.

Danach 15 min bei 42°C und 300 rpm im Thermomixer inkubieren.
Zur Vermeidung von Evaporation die Kavitäten wieder mit der Folie abdecken. Dann Abpipettieren des Hybridisierungspuffers.

Thermomixer auf 53°C einstellen.

2 x waschen mit 50 μl Waschpuffer 1 bei Raumtemperatur. (Zugabe des Waschpuffers 1, kurz mit der Hand schütteln und Puffer durch Invertieren der Mikrotiterplatte entfernen, Mikrotiterplatte auf einer saugfähigen Unterlage leicht ausklopfen).

1 x waschen mit 50 μl Stringency Buffer (Waschpuffer 2) bei Raumtemperatur (kurz mit der Hand schütteln, Puffer durch Invertieren der Mikrotiterplatte und Ausklopfen entfernen).
Der Waschpuffer 2 braucht vor Gebrauch nicht auf 53°C gebracht werden.

Thermomixer auf 21 °C einstellen.

Zugabe von 50 μl Blockierpuffer pro Vertiefung. Inkubation für 10 min bei Raumtemperatur ohne Schütteln.

**Beispiel 4: Detektion**

Entfernen des Blockierpuffers aus den Kavitäten durch Invertieren und leichtes Ausklopfen der Mikrotiterplatte. Verdünnen des Antikörpers in Konjugationspuffer 1:200. Zugabe von 50, μl dieser Lösung pro Kavität.
Inkubation für 30 min bei 300 rpm im Thermomixer bei Raumtemperatur.

Abpipettieren der Antikörperlösung.

3 x waschen mit 50 μl Waschpuffer 3 bei Raumtemperatur.

Entfernen des Waschpuffer 3. Zugabe von 50 μl TMB Substratlösung. Inkubation bei Raumtemperatur im Dunklen ohne Schütteln für 15 min.

Lösung enthält Tetramethylbenzidin (TMB). Hautkontakt vermeiden. Handschuhe tragen.

Zugabe von 50 μl Stopplösung (5% Schwefelsäure) - Farbumschlag von blau nach gelb.

Messung der Absorption im Mikrotiterplattenreader bei 450 nm, Referenzwellenlänge 620 nm (Bestimmung der Absorption in den Kavitäten: OD 450 nm minus OD 620 nm).

Viele Mikrotiterplattenreader erlauben über Software-Einstellungen die direkte Ausgabe der Absorptionsdifferenz ($OD_{450}$ - $OD_{620}$).

Die Messung sollte direkt nach dem Abstoppen der Reaktion erfolgen!

### Beispiel 5: Nachweisgrenzen in Fleischgemischen, Milch, Käse und Futtermitteln

Die folgenden Bestimmungen wurden gemäß den Beispielen 1 bis 4 durchgeführt. Die OD-Werte sind die optischen Dichten, die mit der jeweiligen tierartspezifischen Sonde erhalten wurden. Die DNA wurde mit einem käuflichen Kit zur DNA-Extraktion aus tierischen Lebensmitteln gewonnen (SureFood-PREP Animal, CONGEN, Biotechnologie GmbH, Berlin).

| Fleisch-Gemisch | OD (Schwein) | OD (Rind) |
|---|---|---|
| Rind-Schwein **99,9:0,1%** | 0,21 | 2,22 |
| Rind-Schwein **99:1%** | 0,43 | 2,18. |
| Rind-Schwein **90:10%** | 2,25 | 2,36 |
| Schwein **100%** | 2,39 | |
| Schwein-Rind **99,9:0,1%** | 2,12. | 0,24 |
| Schwein-Rind **99:1%** | 2,32. | 0,84 |
| | 2,08 | 2,15 |
| Rind **100%** | - | 2,32 |
| Schaf-Schwein **99,9:0,1%** | 0,33 | - |
| Schaf-Schwein **99:1%** | 0,72 | - |
| Schaf-Schwein **90:10%** | 2,38 | - |
| Schwein **100%** | 2,46 | - |
| Schaf-Rind **99,9:0,1%** | - | 0,20 |
| Schaf-Rind **99:1%** | - | 0,45 |
| Schaf-Rind **90:10%** | - | 2,80 |
| Rind 100% | - | 3,05 |
| no template control | 0,05 | 0,05 |

Meßergebnisse in Futtermitteln:

| Futtermittel | OD (Schwein) | OD (Rind) | OD (Rind+Schwein) |
|---|---|---|---|
| Futtermittel mit 5% Tiermehl | 1,30 | 1,45 | 2,12 |
| Futtermittel mit 1% Tiermehl | 0,52 | 0,59 | 1,12 |
| Futtermittel mit 0,5% Tiermehl | 0,25 | 0,34 | 0,58 |
| Futtermittel ohne Tiermehlzusatz | 0,03 | 0,04 | 0,06 |

Meßergebnisse in Milchgemischen:

| Milchgemisch | OD (Rind) | OD (Schaf) | OD (Ziege) |
|---|---|---|---|
| Milch (Rind 40%/ Schaf 30%/ Ziege 30%) | 2,02 | 1,92 | 2,10 |
| Milch (Rind 99%/ Schaf 1%) | - | 0,32 | - |
| Milch (Rind 99%/ Ziege 1%) | - | - | 0,35 |
| Milch (Rind 1%1 Schaf 99%) | 0,45 | - | - |
| Milch (Rind 1%I Ziege 99%) | 0,52 | - | - |
| Milch (Schaf 1%/ Ziege 99%) | - | 0,28 | - |
| Milch (Schaf 99%/ Ziege 1%) | | | 0,52 |
| no template control | 0,04 | 0,05 | 0,04 |

**Meßergebnisse in Hartkäsegemischen:**

| Käsegemisch | OD (Rind) | OD (Schaf) | OD (Ziege) |
|---|---|---|---|
| Käse (Rind 90%/ Schaf 5%/ Ziege 5%) | 2,42 | 0,93 | 0,85 |
| Käse (Rind 99%/ Schaf 1%) | - | 0,21 | - |
| Käse (Rind 99%/ Ziege 1%) | - | - | 0,31 |
| Käse (Rind 1%/ Schaf 99%) | 0,59 | - | - |
| Käse (Rind 1%/ Ziege 99%) | 0,31 | - | - |
| Käse (Schaf 1%/Ziege 99%) | - | 0,22 | - |
| Käse (Schaf 99%/ Ziege 1%) | | | 0,62 |
| No template control | 0,05 | 0,04 | 0,04 |

weitere Fleischgemische:

| Fleischgemisch | OD (Schwein) | OD (Rind) |
|---|---|---|
| Lamm:Schwein= 99,9:0,1% | 0,73 | - |
| Lamm:Schwein= 99:1% | 0,87 | - |
| Lamm:Schwein= 90:10 | 2,31 | - |
| Schein: 100 % | 2,61 | - |
| Lamm:Rind = 99,9:0,1% | - | 0,2 |
| Lamm:Rind = 99:1 1 | - | 0,68 |
| Lamm:Rind = 90:10 | - | 2,80 |
| Rind 100% - | | 3,05 |
| Wasser | 0,31 | 0,13 |

**Beispiel 6: Bestandteile eines Kits**

Ein Kit kann die oben genannten Lösungen und Puffer, den in Beispiel 1 genannten PCR-Mix sowie die folgenden Sonden und Kontroll DNAs enthalten.

| Kit-Code | Hybridisierun gssonden | Beschreibung | Lagerung |
|---|---|---|---|
| AXX* | *ida*-probe | Sonde zur Detektion | -20°C |

(fortgesetzt)

| Kit-Code | Hybridisierun gssonden | Beschreibung | Lagerung |
|---|---|---|---|
| | | einer Tierart | |
| B | hyb-probe minus | Sonde zur Kontrolle der Hybridisierungsreaktion | -20°C |
| C | hyb-probe plus | Modifizierte Sonde zur Kontrolle der Hybridisierungsreaktion | -20°C |

| Kit-Code | Kontrol-DNA | Beschreibung | Lagerung |
|---|---|---|---|
| D | positive control DNA | Nachweisgen, Positivkontrolle für die nachzuweisende Tierart | -20°C |
| E | no-template control | Negativkontrolle für die PCR | -20°C |
| F | hyb-control | Kontrolle der Hybridisierungsreaktion | -20°C |

**Patentansprüche**

1. Verfahren zur Bestimmung der Anwesenheit oder Abwesenheit von tierartspezifischer DNA in einer Probe, wobei das Verfahren folgende Schritte umfasst:

   (a) Bereitstellen von für die Zwecke einer Polymerase-Kettenreaktion (PCR) geeigneter DNA aus der Probe,
   (b) Durchführen einer PCR Reaktion mit der DNA von Schritt (a) unter Verwendung eines markierte Primer umfassenden Primergemischs zur Herstellung eines PCR-Produkts, wobei die Primer mit konservierten Bereichen eines in den zu bestimmenden Tieren ubiquitären Gens hybridisieren und wobei das PCR-Produkt einen über alle interessierenden Tierarten oder Tierrassen variablen Bereich mit einer tierartspezifischen Sequenz aufweist,
   (c) Immobilisierung des markierte DNA-Sequenzen enthaltenden PCR-Produkts aus Schritt (b)
   (d) Herstellung von immobilisierter Einzelstrang-DNA des immobilisierten PCR-Produkts aus Schritt (c),
   (e) Behandlung der immobilisierten Einzelstrang-DNA aus Schritt (d) unter Hybridisierungsbedingungen mit mindestens einer tierartspezifischen Sonde,
   (f) Detektion einer Hybridisierung in Schritt (e) zur Bestimmung der Anwesenheit oder Abwesenheit der tierspezifischen DNA,

   wobei das ubiquitäre Gen für mitochondriales Cytochrom b codiert.

2. Das Verfahren gemäß Anspruch 1, wobei die Immobilisierung von Schritt (c) in einem oder mehreren die Markierung der Primer bindenden Flächen- oder Volumenelementen erfolgt.

3. Verfahren nach einem der Ansprüche 1 oder 2, wobei die Anwesenheit oder Abwesenheit von DNA von mindestens zwei Tierarten in einer Probe parallel bestimmt wird, indem in Schritt (e) immobilisierte Einzelstrang-DNA des PCR-Produkts der Probe mit mindestens zwei Sonden, die für verschiedene Tierarten spezifisch sind, behandelt wird, wobei die Behandlung des PCR-Produkts mit den für verschiedene Tierarten spezifischen Sonden auf verschiedenen Flächen- oder Volumenelementen erfolgt.

4. Verfahren nach einem der Ansprüche 1 bis 3 **dadurch gekennzeichnet, dass** die Hybridisierung in Schritt (e) und/ oder in Schritt (f) in Gegenwart eines die Selektivität der Hybridisierung erhöhenden Salzes durchgeführt wird.

5. Verfahren nach Anspruch 4, wobei das die Selektivität der Hybridisierung erhöhende Salz ein Tetraalkylammoniumsalz, insbesondere ein Tetramethylammoniumsalz, ist.

6. Verfahren nach Anspruch 5 **dadurch gekennzeichnet, dass** das Salz ein Chlorid ist.

7. Verfahren nach einem der Ansprüche 1 bis 6, wobei die zu bestimmenden Tierarten Vögel, Fische oder Säugetiere, insbesondere Rind oder Schwein, sind.

8. Verfahren nach einem der Ansprüche 1 bis 7, wobei die tierartspezifische Sonde auf den variablen Bereich des PCR-Produkts gerichtet wird.

9. Verfahren nach Anspruch 1, **dadurch gekennzeichnet, dass** das Primergemisch mindestens einen Primer enthält, der mit dem Sequenzbereich von Position 646 bis 665 des Schweinegens für Cytochrom b gemäß Fig. 2 für PCR-Zwecke hinreichend hybridisiert.

10. Verfahren nach Anspruche 1, **dadurch gekennzeichnet, dass** das Primergemisch mindestens einen Primer enthält, der mit dem Sequenzbereich von Position 751 bis 769 des Schweinegens für Cytochrom b gemäß Fig. 2 für PCR-Zwecke hinreichend hybridisiert.

11. Verfahren nach Anspruch 1, wobei das markierte Primer umfassende Primergemisch mindestens einen der folgenden Primer enthält:

5'-gac aaa att cca ttY cac cc-3', wobei Y für c oder t steht,
5'-tgt agt tgt ctg ggt ctc c-3',
5'-tgt agt tgt ctg ggt ccc c-3'.

12. Verfahren nach einem der Ansprüche 1, 10 oder 11, wobei in Schritt (e) eine tierartspezifische Sonde mit einer der folgenden Sequenzen (in 5'-3' Richtung) benutzt wird:

| | |
|---|---|
| Putensonde: | aat cat act aac ccc ctt a |
| Hirschsonde: | atc tta ctt cta gta ctc ttc |
| Schweinesonde: | ata cta atc cta cta atc c |
| Rindsonde: | cct ctt act aat tct agc |
| Huhnsonde: | tct cat act cac ccc att c |
| Schafsonde 1 | tgc tat cct act aat cct ca |
| Schafsonde 2: | cct cat gct act agt act |
| Entensonde: | tgt cct agg gtt tat tct t |
| Ganssonde: | ctt aat aat cac ccc tct a |
| Pekingente: | tcc tca tgc tta ccc cc. |

13. Verfahren nach einem der Ansprüche 1 bis 12, wobei die Markierung der Primer eine Biotinylierung ist und die Immobilisierung des Schrittes (c) an einen Träger durch Streptavidin erfolgt.

14. Verfahren nach einem der Ansprüche 1 bis 13, wobei die Sonden mit Fluorescinisothiocyanat markiert sind.

15. Verfahren nach einem der Ansprüche 1 bis 14, wobei die Immobilisierung in den Vertiefungen einer Titerplatte erfolgt.

16. Verfahren nach einem der Ansprüche 1 bis 15, wobei zur Detektion in Schritt (f) eine von einer Peroxidase katalysierte Farb- oder Lichtreaktion benutzt wird und wobei die Peroxidase über eine Antigen-Antikörper Bindung an die tierartspezifische Sonde gekoppelt wird.

17. Kit zur Durchführung des Verfahrens gemäß einem der Ansprüche 1 bis 16, umfassend ein markierte Primer enthaltendes Primergemisch und für Tierarten und/oder Gruppen von Tierarten spezifische Sonden, deren Hybridisierung detektiert werden kann, wobei die Primer mit mitochondrialem Cytochrom b Gen hybridisieren.

18. Kit nach Anspruch 17, der weiterhin einen Puffer mit einem die Selektivität der Hybridisierung erhöhenden Salz enthält.

**19.** PCR-Primer, der mit dem Sequenzbereich der Position 646 bis 665 des mitochondrialen Schweinegens für Cytochrom b gemäß Fig. 2 in einem für PCR-Zwecke hinreichendem Maße hybridisiert, wobei der PCR-Primer eine Markierung aufweist, die eine Immobilisierung auf der Oberfläche eines Trägers erlaubt.

**20.** PCR-Primer, der mit dem Sequenzbereich der Position 646 bis 665 des mitochondrialen Schweinegens für Cytochrom b gemäß Fig. 2 in einem für PCR-Zwecke hinreichendem Maße hybridisiert, **gekennzeichnet durch** eine der folgenden Sequenzen:

> 5'-gac aaa att cca ttt cac cc-3',
> 5'-gac aaa att cca ttc cac cc-3'.

**21.** Der PCR-Primer nach Anspruch 20, **gekennzeichnet durch** eine Markierung.

**22.** PCR-Primer, der mit dem Sequenzbereich der Position 751 bis 769 des mitochondrialen Schweinegens für Cytochrom b gemäß Fig. 2 in einem für PCR-Zwecke hinreichendem Maße hybridisiert.

**23.** PCR-Primer nach Anspruch 22 **gekennzeichnet durch** eine der folgenden Sequenzen:

> 5'-tgt agt tgt ctg ggt ctc c-3',
> 5'-tgt agt tgt ctg ggt ccc c-3'.

**24.** PCR-Primer nach einem der Ansprüche 22 oder 23, **gekennzeichnet durch** eine Markierung.

**25.** Tierartspezifische markierte Sonde, welche mit der in Anspruch 1 definierten tierartspezifischen Sequenz in einem für die selektive Detektion hinreichenden Maß hybridisiert, wobei der variable Bereich der tierartspezifischen Sequenz von der Position 666 bis zur Position 750 bezogen auf das Cytochrom b Gen des Schweins gemäß Fig. 2 als Referenzsequenz reicht.

**26.** Sonde nach Anspruch 25, **gekennzeichnet durch** eine der folgenden Sequenzen (in 5'-3'-Richtung):

> aat cat act aac ccc ctt a,
> atc tta ctt cta gta ctc ttc,
> ata cta atc cta cta atc c,
> cct ctt act aat tct agc,
> tct cat act cac ccc att c,
> tgc tat cct act aat cct ca,
> cct cat gct act agt act,
> tgt cct agg gtt tat tct t,
> ctt aat aat cac ccc tct a,
> tcc tca tgc tta ccc cc.

**Claims**

**1.** Process for determining the presence or absence of animal species-specific DNA in a sample, the process comprising the following steps:

> (a) providing, from the sample, DNA that is suitable for polymerase chain reaction (PCR),
> (b) conducting a PCR reaction using the DNA of step (a), wherein a mixture of primers is used comprising labeled primers for producing a PCR product, wherein the primers hybridize to conserved sequence ranges of a gene that is ubiquitous to the animals to be determined, and wherein the PCR product comprises a region being variable over all animal species or animal races of interest and having an animal species-specific sequence,
> (c) immobilizing the PCR product from step (b) comprising labeled DNA sequences,
> (d) providing immobilized single stranded DNA of the immobilized PCR product from step (c),
> (e) treating the immobilized single stranded DNA from step (d) with at least one animal species-specific probe using conditions suitable for hybridization,

(f) detection of a hybridization in step (e) for determining the presence or absence of the animal species-specific DNA,

wherein the ubiquitous gene codes for mitochondrial cytochrome b.

2. The process according to claim 1, wherein the immobilization of step (c) occurs in one or more area elements or space elements that bind the labeling of the primers.

3. The process according to any of claims 1 or 2, wherein the presence or absence of DNA from at least two animal species in a sample is determined in parallel, in that the single stranded DNA of the PCR product of the sample immobilized in step (e) is treated with at least two probes that are specific for different animal species and wherein the treatment of the PCR product with the probes specific for different animal species is conducted on different area or space elements.

4. The process according to any of claims 1 to 3, **characterized in that** the hybridization in step (e) and/or in step (f) is carried out in the presence of a salt that increases the selectivity of the hybridization.

5. The process according to claim 4, wherein the salt that increases the selectivity of the hybridization is a tetraalkylammonium salt, preferably a tetramethylammonium salt.

6. The process according to claim 5, **characterized in that** the salt is a chloride.

7. Process according to any one of claims 1 to 6, wherein the animal species to be determined are birds, fish or mammals, preferably cattle or pigs.

8. The process according to any one of claims 1 to 7, wherein the animal species specific probe is directed to the variable region of the PCR product.

9. The process according to claim 1, **characterized in that** the mixture of primers contains at least one primer that hybridizes sufficiently for PCR purposes with the sequence region of from position 646 to 665 of the pig gene for cytochrome b according to Fig. 2.

10. The process according to claim 1, **characterized in that** the mixture of primers contains at least one primer that hybridizes sufficiently for PCR purposes with a sequence region of position 751 to 769 of the pig gene for cytochrome b according to Fig. 2.

11. The process according to claim 1, wherein the mixture of primers comprising labeled primers contains at least one of the following primers:

   5'-gac aaa att cca ttY cac cc-3', wherein Y stands for c or t,
   5'-tgt agt tgt ctg ggt ctc c-3',
   5'-tgt agt tgt ctg ggt ccc c-3'.

12. The process according to any one of claims 1, 10 or 11, wherein in step (e) an animal species-specific probe is used having one of the following sequences (in 5'-3' direction):

| | |
|---|---|
| turkey probe: | aat cat act aac ccc ctt a |
| deer probe: | atc tta ctt cta gta ctc ttc |
| pig probe: | ata cta atc cta cta atc c |
| cattle probe: | cct ctt act aat tct agc |
| chicken probe: | tct cat act cac ccc att c |
| sheep probe 1: | tgc tat cct act aat cct ca |
| sheep probe 2: | cct cat gct act agt act |
| duck probe: | tgt cct agg gtt tat tct t |
| goose probe: | ctt aat aat cac ccc tct a |
| Pekin duck probe: | tcc tca tgc tta ccc cc. |

**13.** The process according to any one of claims 1 to 12, wherein the labeling of the primers is a biotinylation and the immobilization of step (c) to a carrier is through streptavidin.

**14.** The process according to any one of claims 1 to 13, wherein the probes are labeled with fluorescinisothiocyanate.

**15.** The process according to any one of claims 1 to 14, wherein the immobilization is carried out in the recesses of a tissue culture plate.

**16.** The process according to any one of claims 1 to 15, wherein a color or light reaction catalyzed by a peroxidase is used for the detection of step (f) and wherein the peroxidase is linked to the animal species-specific probe via an antigen-antibody bond.

**17.** Kit for performing the process according to any one of claims 1 to 16, comprising

- a mixture of primers comprising labeled primers and
- probes that are specific for animal species and/or for groups of animal species, hybridization of said probes being detectable,

whereby the primers hybridize with mitochondrial cytochrome b gene.

**18.** The kit according to claim 17, further comprising a buffer having a salt that increases the selectivity of hybridization.

**19.** PCR primer that hybridizes sufficiently for PCR purposes with the sequence region of from position 646 to 665 of the mitochondrial cytochrome b gene from pig according to Fig. 2, said PCR primer comprising a label allowing immobilization on the surface of a carrier.

**20.** PCR primer that hybridizes sufficiently for PCR purposes with position 646 to 665 of the mitochondrial cytochrome b gene from pig according to Fig. 2, **characterized by** one of the following sequences:

5'-gac aaa att cca ttt cac cc-3',
5'-gac aaa att cca ttc cac cc-3'.

**21.** The PCR primer according to claim 20, **characterized by** a label.

**22.** PCR primer that hybridizes sufficiently for PCR purposes with the sequence region of position 751 to 769 of the mitochondrial cytochrome b gene from pig according to Fig. 2.

**23.** The PCR primer according to claim 22, **characterized by** one of the following sequences:

5'-tgt agt tgt ctg ggt ctc c-3',
5'-tgt agt tgt ctg ggt ccc c-3'.

**24.** PCR primer according to claim 22 or 23, **characterized by** a label.

**25.** Animal species-specific labeled probe that hybridizes with the animal species-specific sequence defined in claim 1 to a degree sufficient for selective detection, wherein the variable region of the animal species-specific sequence ranges from position 666 to position 750 relative to the cytochrome b gene from pig according to Fig. 2 as a reference sequence.

**26.** The probe according to claim 25, **characterized by** one of the following sequences (in 5'-3' direction):

aat cat act aac ccc ctt a,
atc tta ctt cta gta ctc ttc,
ata cta atc cta cta atc c,
cct ctt act aat tct agc,
tct cat act cac ccc att c,
tgc tat cct act aat cct ca,
cct cat gct act agt act,

tgt cct agg gtt tat tct t,
ctt aat aat cac ccc tct a,
tcc tca tgc tta ccc cc.

**Revendications**

1. Procédé de détermination de la présence ou de l'absence d'ADN spécifique d'une espèce animale dans un échantillon, le procédé comprenant les étapes suivantes :

   (a) mise à disposition d'un ADN de l'échantillon, qui est approprié pour la réalisation d'une réaction en chaîne par polymérase (PCR),
   (b) réalisation d'une réaction de PCR avec l'ADN de l'étape (a) en utilisant un mélange d'amorces comprenant une amorce marquée, pour produire un produit de PCR, l'amorce s'hybridant avec des domaines conservés d'un gène ubiquitaire dans les animaux à déterminer, le produit de PCR présentant un domaine variable sur toutes les espèces ou races animales intéressantes avec une séquence spécifique d'une espèce d'animal,
   (c) immobilisation du produit de PCR contenant des séquences d'ADN marquées, de l'étape (b),
   (d) production d'ADN à brin simple immobilisé du produit de PCR immobilisé de l'étape (c),
   (e) traitement de l'ADN à brin simple immobilisé de l'étape (d) dans des conditions d'hybridation avec au moins une sonde spécifique d'une espèce animale,
   (f) détection d'une hybridation à l'étape (e) pour déterminer la présence ou l'absence de l'ADN spécifique d'un animal,

   le gène ubiquitaire codant pour le cytochrome b mitochondrial.

2. Procédé selon la revendication 1, dans lequel l'immobilisation de l'étape (c) a lieu dans un ou plusieurs éléments de surface ou de volume se liant au marquage des amorces.

3. Procédé selon l'une des revendications 1 et 2, dans lequel la présence ou l'absence de l'ADN d'au moins deux espèces animales est déterminée en parallèle dans un échantillon, en traitant à l'étape (e), l'ADN à brin simple immobilisé du produit de PCR de la sonde avec au moins deux sondes qui sont spécifiques des différentes espèces animales, le traitement du produit de PCR s'effectuant avec les sondes spécifiques des différentes espèces animales sur différents éléments de surface ou de volume.

4. Procédé selon l'une des revendications 1 à 3, **caractérisé en ce que** l'hybridation est réalisée à l'étape (e) et/ou à l'étape (f) en présence d'un sel augmentant la sélectivité de l'hybridation.

5. Procédé selon la revendication 4, dans lequel le sel augmentant la sélectivité de l'hybridation est un sel de tétra-alkylammonium, en particulier un sel de tétraméthylammonium.

6. Procédé selon la revendication 5, **caractérisé en ce que** le sel est un chlorure.

7. Procédé selon l'une des revendications 1 à 6, dans lequel les espèces animales à déterminer sont des oiseaux, des poissons ou des mammifères, en particulier un boeuf ou un cochon.

8. Procédé selon l'une des revendications 1 à 7, dans lequel la sonde spécifique de l'espèce animale cible le domaine variable du produit de PCR.

9. Procédé selon la revendication 1, **caractérisé en ce que** le mélange d'amorces contient au moins une amorce qui s'hybride suffisamment avec le domaine de séquence de la position 646 à la position 665 du gène de cochon pour le cytochrome b selon la figure 2 pour réaliser la PCR.

10. Procédé selon la revendication 1, **caractérisé en ce que** le mélange d'amorces contient au moins une amorce qui s'hybride suffisamment avec le domaine de séquence de la position 751 à la position 769 du gène de cochon pour le cytochrome b selon la figure 2, pour réaliser la PCR.

11. Procédé selon la revendication 1, dans lequel le mélange d'amorces comprenant l'amorce marquée contient au moins l'une des amorces suivantes :

5'-gac aaa att cca ttY cac cc-3', Y désignant c ou t,
5'-tgt agt tgt ctg ggt ctc c-3',
5'-tgt agt tgt ctg ggt ccc c-3'.

**12.** Procédé selon l'une des revendications 1, 10 et 11, dans lequel à l'étape (e), une sonde spécifique d'une espèce animale est utilisée avec les séquences suivantes (dans le sens 5'-3') :

Sonde de dinde : aat cat act aac ccc ctt a
Sonde de cerf : atc tta ctt cta gta ctc ttc
Sonde de cochon : ata cta atc cta cta atc c
Sonde de boeuf : cct ctt act aat tct agc
Sonde de poule : tct cat act cac ccc att c
Sonde de mouton 1 : tgc tat cct act aat cct ca
Sonde de mouton 2 : cct cat gct act agt act
Sonde de canard : tgt cct agg gtt tat tct t
Sonde d'oie : ctt aat aat cac ccc tct a
Sonde de canard de Pékin : tcc tca tgc tta ccc cc.

**13.** Procédé selon l'une des revendications 1 à 12, dans lequel le marquage de l'amorce est une biotinylation et l'immobilisation de l'étape (c) s'effectue sur un support par la streptavidine.

**14.** Procédé selon l'une des revendications 1 à 13, dans lequel les sondes sont marquées avec de l'isothiocyanate de fluorescéine.

**15.** Procédé selon l'une des revendications 1 à 14, dans lequel l'immobilisation s'effectue dans les creux d'une plaque de titrage.

**16.** Procédé selon l'une des revendications 1 à 15, dans lequel, pour la détection à l'étape (f), est utilisée une réaction colorée ou lumineuse catalysée par la peroxydase et la peroxydase étant couplée par une liaison antigène-anticorps sur la sonde spécifique d'une espèce animale.

**17.** Kit pour la mise en oeuvre du procédé selon l'une des revendications 1 à 16, comprenant un mélange d'amorces contenant une amorce marquée et des sondes spécifiques des espèces animales et/ou des groupes d'espèces animales, dont l'hybridation peut être détectée, les amorces s'hybridant avec le gène du cytochrome b mitochondrial.

**18.** Kit selon la revendication 17, qui contient en outre un tampon avec un sel augmentant la sélectivité de l'hybridation.

**19.** Amorce de PCR qui s'hybride avec le domaine de séquence de la position 646 à la position 665 du gène mitochondrial de cochon pour le cytochrome b selon la figure 2, dans une proportion suffisante pour réaliser la PCR, l'amorce de PCR présentant un marquage qui permet une immobilisation sur la surface d'un support.

**20.** Amorce de PCR qui s'hybride avec le domaine de séquence de la position 646 à la position 665 du gène mitochondrial de cochon pour le cytochrome b selon la figure 2, dans une proportion suffisante pour réaliser la PCR, **caractérisée par** l'une des séquences suivantes :

5'-gac aaa att cca ttt cac cc-3'
5'-gac aaa att cca ttc cac cc-3'.

**21.** Amorce de PCR selon la revendication 20, **caractérisée par** un marquage.

**22.** Amorce de PCR qui s'hybride avec le domaine de séquence de la position 751 à la position 769 du gène mitochondrial de cochon pour le cytochrome b selon la figure 2 dans une proportion suffisante pour réaliser la PCR.

**23.** Amorce de PCR selon la revendication 22, **caractérisée par** l'une des séquences suivantes :

5'-tgt agt tgt ctg ggt ctc c-3'
5'-tgt agt tgt ctg ggt ccc c-3'.

**24.** Amorce de PCR selon l'une des revendications 22 et 23, **caractérisée par** un marquage.

**25.** Sonde marquée spécifique d'une espèce animale, qui s'hybride à la séquence spécifique d'une espèce animale, définie à la revendication 1, dans une proportion suffisante pour la détection sélective, le domaine variable de la séquence spécifique d'une espèce animale allant de la position 666 à la position 750 par rapport au gène du cytochrome b du cochon selon la figure 2 ; comme séquence de référence.

**26.** Sonde selon la revendication 25, **caractérisée par** l'une des séquences suivantes (dans le sens 5'-3') :

      aat cat act aac ccc ctt a,
      atc tta ctt cta gta ctc ttc,
      ata cta atc cta cta atc c,
      cct ctt act aat tct agc,
      tct cat act cac ccc att c,
      tgc tat cct act aat cct ca,
      cct cat gct act agt act,
      tgt cct agg gtt tat tct t,
      ctt aat aat cac ccc tct a,
      tcc tca tgc tta ccc cc.

EP 1 364 068 B1

Gereinigte DNA
aus Probe

DNA-Amplifizierung
eines kurzen Fragments
mit einem biotinylierten
Primer

PCR

Binden
auf Streptavidin-beschichteter Mikrotiterplatte

Denaturierung
mit alkalischer Lösung

Hybridisierung
mit markierter Sonde

Detektion
Binden der hybridisierten Sonde durch einen
Antikörper

Kolorimetrische
Messung
nach enzymatischer
Reaktion

Fig 1

24

```
CYTB_SCH   ATGACCAACATCCGAAAATC...ACACCCACTAATAAAAATTATCAACAACGCATTCATT   57
CYTB_HSC   --------------------...------------------------------------   57
CYTB_RIN   -----t-----t-----g--...c-----------------g-a-----+t--------c   57
CYTB_HIR   ----t---t-c-------a-...c------t-----------g-a------------t---   57
CYTB_REH   --------t---------a-...t-----------------g-a--t------------   57
CYTB_SHF   ----------------a-...c-----------------g-a-------------   57
CYTB_ZIE   ----------------ga-...c------t-----------g-a------------t---   57
CYTB_PFE   -----a--------g-----...t-----------t-----c-----tc--t-t--t---   57
CYTB_HUN   -----------t------a-...c---------gcc-----g-t--t---t--------   57
CYTB_PUT   ---g-acc--atatccg-aaatg------c---t------ca---------t----a--c   60
CYTB_HHU   ---g-acc--a-attcg-aaatcc-----c---c------a-t------t-cc-a--c   60
CYTB_VO1   ------cc--a--t-cgtaaaaac--------cc------cg-a---g--t-cc-g--c   60
CYTB_KAN   -----------t--c---a-...c-----c---t-------g-t---c-t-cc-a---   57
CYTB_KAT   -----------t---------...-----c--t--c--------t--tc--t-------c   57
CYTB_LAC   ---g--...-a--tccg-aaaact----g--cc-------gct--tg-----c-ag-c   57
CYTB_AAL   ---g-a...-a--t-cg-aaaacc-------cc-------gct---g-t--cc-ag-g   57


CYTB_SCH   GACCTCCCAGCCCCCTCAAACATCTCATCATGATGAAACTTCGGTTCCCTCTTAGGCATC   117
CYTB_HSC   -----------------------------------------------------------   117
CYTB_RIN   -----t---------a--------t---------------t-----------c-g--a---   117
CYTB_HIR   --------------a--g--t--t-----c---------t-----c--t-ac----a--t   117
CYTB_REH   --t-----------a----t--------------------t-----t--a-----a---   117
CYTB_SHF   ----------t--a----t--t------------------t--c-----c------t   117
CYTB_ZIE   --------a----a-------------------------t--a-----c-----a--t   117
CYTB_PFE   -----a-------------t--------------------c-----c---a---   117
CYTB_HUN   ----------g--g--t--------tg-t-----g--------a---t-ac----ag-a   117
CYTB_PUT   ---------a----a--c--------cg-t-----------c-----ac---cag-a   120
CYTB_HHU   ----------a--c--------tg-t--------t-----c-----a----cag--   120
CYTB_VO1   -----a---a-t--a-----------g-c-----------t--g-----c--------   120
CYTB_KAN   -----t--t--t--a-------------tg-c-----------t--c--t--ac-----c-g   117
CYTB_KAT   --t--a--ca----a--t---------g--------------c-----tc----ag--   117
CYTB_LAC   --t--------a--a--t--------gtt-----------t--c--a---------c-a   117
CYTB_AAL   --t--a---a-------c-----t--g----------t--t--c--t---c----at-a   117


CYTB_SCH   TGCCTAATCTTGCAAATCCTAACAGGCCTGTTCTTAGCAATACATTACACATCAGACACA   177
CYTB_HSC   -----------------------------------------------------------   177
CYTB_RIN   ---------c-a-------c-------a---c----------c--------c------   177
CYTB_HIR   --t------c-a-----t--c--------a---c----------c--t-----c--t---   177
CYTB_REH   --t--------a-------c-------a---c----------c-------c------   177
CYTB_SHF   ---t----t--a--g--t----------a---c----------c--t---c-c------   177
CYTB_ZIE   -----------a--------g--------a--c----------c--t-----c------   177
CYTB_PFE   ---------c-c-----t---------a---c----c-----c------------g   177
CYTB_HUN   ---t-g--tc-a--g--t--------tt-a--------t--g--c--t-----g-----   177
CYTB_PUT   -----c---act------t----c-----cc-ac---c-----------tg-------c   180
CYTB_HHU   -----c--gacc--------c--c-----ac-ac---c--g--c-----g--------   180
CYTB_VO1   ---------aca------g-c--------cc-tc----c-----c-----cg-------c   180
CYTB_KAN   -----t--aa-t-----tt-c--t-----a--------c-----c-----c--t------   177
CYTB_KAT   -----------a--------c--c-----c--t--g--c-----c------------   177
CYTB_LAC   --t---gc-acc--------t--c--g--c---c---c-----c-----c--c--t-tc   177
CYTB_AAL   -----t----ca--------t-----a--a---c---c-----c------------tc   177
```

Fig 2

25

```
CYTB_SCH   ACAACAGCTTTCTCATCAGTTACACACATTTGTCGAGACGTAAATTACGGATGAGTTATT   237
CYTB_HSC   ------------------------------------------------------------   237
CYTB_RIN   --------a------c--t-----c--t--c--c---------g--c-----c---a-c--c   237
CYTB_HIR   -t------a--t--c--t--c--c--t--c--------t--c-----t--t--+a-----   237
CYTB_REH   -t------a-----c--t--c--t-----c--c--------t--c--t--c---a----c   237
CYTB_SHF   --------a-----c--t--a--c--------c---------g--c--t--c---a----c   237
CYTB_ZIE   -t------a--t--c--t--a--t--------------t--------t--c---a-c--c   237
CYTB_PFE   -----t--c--------c--c--t-----c--c--------t--c---------a-----   237
CYTB_HUN   g-c--------t--------c--c-----c--c--------t--c-----c---a----c   237
CYTB_PUT   --tctt--a-----t--t--gg-ct---ca--c---a-----c-a-----t---c-cc-c   240
CYTB_HHU   t-cct---c-----c--c--ag-c-----c---c--ga----c-a-----c---c-c--c   240
CYTB_VO1   t-cct---a---aac-----ag-c--t--a--c---a-----c-g-t-------c-a--c   240
CYTB_KAN   --------a---------a--c--t-----c-----t--t--c--t--c---c-----c   237
CYTB_KAT   -t---c--c--t---------c-----c-----c-----t-----t--c---a-c--c   237
CYTB_LAC   t---------t--c--t---tgc--------c-----t--t-gc--t--c---c-c--c   237
CYTB_AAL   t----t--c--t--c----ag-c-----c--c--------t-----t-------t-g--c   237


CYTB_SCH   CGCTATCTACATGCAAACGGAGCATCCATATTCTTTATTTGCCTATTCATCCACGTAGGC   297
CYTB_HSC   ------------------------------------------------------------   297
CYTB_RIN   --a--ca----c-----------t--a--g--t-----c---t---at--g-------a   297
CYTB_HIR   --a--ca----c--------g-----a-----t--c--c---------a--t-----a   297
CYTB_REH   --a--a---------------------a-----t-----c---t-----c-a--t-----a   297
CYTB_SHF   --a--a----c--------g-----a-----t-----c-------t--g--t-----a   297
CYTB_ZIE   --a--ca----c--------------a-----------c-----------a--ta-c--a   297
CYTB_PFE   -----c--c-----c-----------a-----t-----c-----c-----t-------a   297
CYTB_HUN   ------a-g--c-----t--c--t--------------c---------c-a--t-----a   297
CYTB_PUT   -ata-c--c-----g--t--g--c--at-c-----c--c---a-c---c-a---a-t--a   300
CYTB_HHU   --ga----c--c--------c--c--at-c-----c--c--ta-c---c-t---a-c--a   300
CYTB_VO1   --aa------c-----------c--tt-c-----c--c---a-c-a-c-a--ta-c---   300
CYTB_KAN   --a--c--c--c--t----------t--------------c-a---a---------   297
CYTB_KAT   --a---t----c--c-------t--t-----------c-----g-a---a--t-----a   297
CYTB_LAC   --ta-ca-t--c--t----------tt-c-------c--ta-t-at--a---a-c-c-   297
CYTB_AAL   ---a-ct-----------------c---t-c----------t--c-a-c-g---a-t-c-   297


CYTB_SCH   CGAGGTCTATACTACGGATCCTATATATTCCTAGAAACATGAAACATTGGAGTAGTCCTA   357
CYTB_HSC   ------------------------------------------------------------   357
CYTB_RIN   -----ct----t-----g--t--c-ct--t---------------t---------a----t   357
CYTB_HIR   -----c----------g--a--c-ct--t-----g-----------c------a----c   357
CYTB_REH   -----c--------t-----t--c-ct--t------------------------a-t--c   357
CYTB_SHF   -----c-----t--t-----a----cc--------------------c------a----c   357
CYTB_ZIE   -----------t--t-----a----cc--t-----------------------a----c   357
CYTB_PFE   --c--c--c--------c--t--c-c---------g--------------a-ca-----   357
CYTB_HUN   -----c-----t-----------g-----a---------------------a-t--a---   357
CYTB_PUT   --c--c-----t--t--t--g--cc---ataa-----c-----t-ca--------t--   360
CYTB_HHU   -----c-----------c-----cc-c-a-aag----c-------ca------a---c   360
CYTB_VO1   -----a-----------c--a--cc--aa-aa-----c------g-a--g---a-----   360
CYTB_KAN   --c--aa-c-----t-----a--c-c--a------g--c-----------ca-ca----c   357
CYTB_KAT   --g--aa-g--------c-----c-cc---tc--g-------------a-ca-a---   357
CYTB_LAC   -----a--t--t--t--t------c---ataa-----c-----t--c-----t--a--t   357
CYTB_AAL   -----a--t--------c--a---c-t-ataa---g----------c-----c--a---   357
```

zu Fig. 2

```
CYTB_SCH   CTATTTACCGTTATAGCAACAGCCTTCATAGGCTACGTCCTGCCCTGAGGACAAATATCA   417
CYTB_HSC   ------------------------------------------------------------   417
CYTB_RIN   --gc-c--a--a-----c-------a--t-----a--------a--a-------------   417
CYTB_HIR   --------a--------c-----a---g----a--t-----a--a---------------   417
CYTB_REH   -----c--a--a-----c--g--a--tg----a-----tt-a--a--------------   417
CYTB_SHF   ------g-gaca-----c-----a-----------t---t-a--a--------------   417
CYTB_ZIE   --gc-cg-gaca--g--c-----a----------t-----a--a--------------   417
CYTB_PFE   --t--c--a--------t-----a----g-----t-----a--a-----c-------c   417
CYTB_HUN   t----cg-aacc-----c-----a-----g-----t--a--a--------------   417
CYTB_PUT   --tc-c---c-c------------tg-------t-----t--a--g-----------   420
CYTB_HHU   --cc-c--ac-c-----c--c-----tg-g-----t--t--c--a--g--c--------   420
CYTB_VO1   ---c-a--c-a--------t-----tg----a--------a--a-----------c   420
CYTB_KAN   -----cg-a--a-----c-----a--t--g--t--t-----c--a-------------   417
CYTB_KAT   t-------a--c-----c-----t--t--g--a--------a--a-----c-----g--c   417
CYTB_LAC   ---c--ct-ac----at---t-----g-----------t--t--a-------------c   417
CYTB_AAL   t-cc-atta--a---at------a---g----g-t--a--t-a-----g--------   417
```

```
CYTB_SCH   TTCTGAGGAGCTACGGTCATCACAAATCTACTATCAGCTATCCCTTATATCGGAACAGAC   477
CYTB_HSC   ------------------------------------------------------------   477
CYTB_RIN   -------------a--a--------c--c--ct-------a-----a--c-----c---a-t   477
CYTB_HIR   -------------a--a-----t--c--c--t--c-----a--t--a--c--t--c---a--   477
CYTB_REH   -------------a--a--t--t--c-----c--c-----a--t--a------t---a--   477
CYTB_SHF   -------------a--a--t--t--c--c--c--t-----a--t--a-----t--c---a--   477
CYTB_ZIE   --t-----g--a--a-------c-----c--c-----a-----a-----t--c---a--   477
CYTB_PFE   --t---------a--a--------g--c--c--------a--t--c--c-----t--tac-   477
CYTB_HUN   --t---------a--t--a-----t-----t--c--t--c--------------t---   477
CYTB_PUT   --------g-----c-----------c---t-c-----a-----c--c------ca-ac-   480
CYTB_HHU   --------g--c--c--t--------c---t-c-----a--t--c--c--t---cacac-   480
CYTB_VO1   --------g-----a--a--t--t--c---t-t-----a-----a--c------ca-aca   480
CYTB_KAN   --t--g-----a--c--a-----t--c--c--------a-----a----------ac-   477
CYTB_KAT   -----------a--c--a-----c--c--c--g-----a--t--a--c-----g--t--a   477
CYTB_LAC   -----------c--t--a--t-----c--c--c--c---g----c--cg-a---ggc-c-   477
CYTB_AAL   --------t-----a--a--t--c--c--------c---g---a---g-a--g-actc-   477
```

```
CYTB_SCH   CTCGTAGAATGAATCTGAGGGGGCTTTTCCGTCGACAAAGCAACCCTCACACGATTCTTC   537
CYTB_HSC   ------------------------------------------------------------   537
CYTB_RIN   t-a--c--------------c--a--c--a--a-------------t--c---------   537
CYTB_HIR   --a--c-----gg-------a--------a--a--t------------a--c---------   537
CYTB_REH   --a--t--------t--------------a--a-----------------g--t-----t---   537
CYTB_SHF   --a--c----------g--a--a--c--a--a--------t--------c-----t---   537
CYTB_ZIE   --a--c-------------------a--c--a--a-------c--t-----c---------   537
CYTB_PFE   -----c--g-----------t--a--c--a--a--------c-----t--c-----t--t   537
CYTB_HUN   t-a--------g--------c-----c--a--g------------a----------t   537
CYTB_PUT   --a-----g---gc---------a--c--a--a-----cc-------t--c---------   540
CYTB_HHU   --a-----g---gc---------a-----a--------cc------t--c---------   540
CYTB_VO1   --t----------gc---------a--c--a--a-----cc----a--g--c-------t   540
CYTB_KAN   t-a--t-------------a--a-----a--t--t-----c--t--t--c---------   537
CYTB_KAT   --a----------------------c--a--a-------c-----a----------t   537
CYTB_LAC   --t---c--------t-----a--a-----t--a-----c--c-----a--------t---   537
CYTB_AAL   --a--cc--------------------c--a--t-----c--c--at-a--c---------   537
```

zu Fig. 2

```
CYTB_SCH  GCCTTCCACTTTATCCTGCCATTCATCATTACCGCCCTCGCAGCCGTACATCTCCTATTC  597
CYTB_HSC  -----t------------------------------------------------------  597
CYTB_RIN  --t-----t--------t-----t-----c-ta--aa-t--cata--c--c--a------  597
CYTB_HIR  --t-----------t--c-----t-----cg-a--a-----tata-----ct-a--c---  597
CYTB_REH  -----t------------c-----t-----g-a--a--t--tata--c---t-a--t---  597
CYTB_SHF  -----t---------tt-c----------cg-a---------cata--t--c--a--c---  597
CYTB_ZIE  ------------------c-----------c--a-g------cata--c--c--g--c---  597
CYTB_PFE  --t--------c-----a--c---------c--a-----g-t--t-------t-a-----t  597
CYTB_HUN  --a-----t--c-----c--t--------cg-a--t--a---ata-----c--------t  597
CYTB_PUT  ---c------cc-------c--g-a--cg-a-gaa-ta--at-a-t--c---a----t  600
CYTB_HHU  --t--a-----cc----c--c--tgca--cg-a-gta-ta-tat-a-c--c---acc--t  600
CYTB_VO1  --tc-g--t--cc----a--t--tg-a--cg-a-ga--ta--cta-----c--aacc---  600
CYTB_KAN  --t--t-----c--t--------t------g-aa-tt-a-tcttaa-t--c-----c--t  597
CYTB_KAT  ----------c--t--t--------t--ct-a---t-a-----a-----c---t-----  597
CYTB_LAC  ----------cc--at-c------g-t---g-a--tgc-a---tac-c--i---t-----t  597
CYTB_AAL  --a--------cc--at-t-----tg-ag--g---g-gcta--ataa-t--c--------  597


CYTB_SCH  CTGCACGAAACCGGATCCAACAACCCTACCGGAATCTCATCAGACATAGACAAAATTCCA  657
CYTB_HSC  ------------------------------------------------------------  657
CYTB_RIN  --c---------a--c-----------a--a-----t--c------g----------c---  657
CYTB_HIR  --t-----g--a------t--t-----a--a-----c-------gc---------c--c  657
CYTB_REH  --c---------a-----a--------g-ta------c-----a--gcg-----------  657
CYTB_SHF  --c--------a--------------c--a-----tc----g----c---t-------c  657
CYTB_ZIE  --c---------a-----g--------c--a-----tc---------c--t-----c---  657
CYTB_PFE  --t---------a-----t--t-----ct-a------c----c--t--g---------c---  657
CYTB_HUN  --a----------------------t-a------a--------tc------------  657
CYTB_PUT  ----------t-a--c--a-----t---ctt--c---------a--gct--------c---  660
CYTB_HHU  --a-------t-a--c--a---------ccta--c--------c---tct-----------  660
CYTB_VO1  --a---------a-----a--------ccta------c--------tgc--------c---  660
CYTB_KAN  --a--t-----t--c-----------c--a-----tc-t---a--tc---t-----c--t  657
CYTB_KAT  --t--t-----a-----t---------ct-a-----ta-----c--ttc--------c---  657
CYTB_LAC  t-a--t---------g--t--t-----ag-a--c---aac--c--tgcc--t-----ct--  657
CYTB_AAL  --t--t-----a-----a---------agta---c-gaac--t--tgc---------c---  657


CYTB_SCH  TTTCACCCATACTACACTATTAAAGACATTCTAGGAGCCTTATTTATAATACTAATCCTA  717
CYTB_HSC  ------------------------------------------------------------  717
CYTB_RIN  --c------c-----t--c-----g-----ct----g---c-c--ac----t---gct---  717
CYTB_HIR  --c------t-----t--g--------t--ct----tat----c--c-------ct-----  717
CYTB_REH  --------c-----t--c---------t--c-------ttc----cc----t--ttc----  717
CYTB_SHF  --c-----t--t-----c-----------c-----c---a--c-ac-----c--tgc---c  717
CYTB_ZIE  --------t---------c--------t--ct-----c---a-gc-ac----t--tg-t---  717
CYTB_PFE  --c--------t--t--a----------c------ct-c-cc-cc-g--ct-gc-----  717
CYTB_HUN  --------t---------a--c--g--t--c-----------c-cc--c-c--------  717
CYTB_PUT  --c-----c-------t----c---------c-----tctaac-a-c---c--acccc-t--  720
CYTB_HHU  ----------------t-ct-c----------g--cttaactc-c---c-caccccat-c  720
CYTB_VO1  --c-----c--------c-c--g--t--c------tt-gccc-a---c--acctct---  720
CYTB_KAN  --c-----c--------a--c-------cc-----ttt-c-tg-agcc--t--tc-----c  717
CYTB_KAT  --c----------t--a--c---------c-----tcttc--g-ac--g-tt---ca--c  717
CYTB_LAC  --c-----t-----t-t-ata-------c-c--c---tttg--gcc---c-----tgg----  717
CYTB_AAL  --c-----------t-t-ctac------c-g------tt-a-ta-----c--accgctt--  717
```

Zu Fig. 2

28

```
CYTB_SCH  CTAATCCTTGTACTATTCTCACCAGACCTACTAGGAGACCCAGACAACTACACCCCAGCA  777
CYTB_HSC  ------------------------------------------------------------  777
CYTB_RIN  a--c-a--a---------g----c-----c--c--------------t---------c  777
CYTB_HIR  a--t-a--a---t-----g-------t--g--t-----------------t--------  777
CYTB_REH  a--t-a--a--c-----g-------g--t---------------t-------a-----  777
CYTB_SHF  a-gc-a--a---------a---t---t----c-----------t----t--------  777
CYTB_ZIE  a--c-a--a---------a---c------c---------------t-t-------  777
CYTB_PFE  ----ct-a---t-------c--c-----c----------------------t  777
CYTB_HUN  a--tca--a--tt----t---t------t----------------t---------t---  777
CYTB_PUT  --c-cat-aacc-----------ta----ct--------------a----tt--------  780
CYTB_HHU  ----ca---a-cc--------c--ca----c--------------a---t---------  780
CYTB_VO1  g--gc---a-cc--------c---a----c--------------a--t-t---g--c--c  780
CYTB_KAN  --t--tt-a--c-----t-----------t--------------------------t--c  777
CYTB_KAT  ac-c-a--c--c--r--t---------g---------------------t-----c  777
CYTB_LAC  ac-tc-t-a-ct-----g----ca----c--c--g-----------t-tt--a--t--c  777
CYTB_AAL  ac---a----cc--------ac---a----c--t-----------------tt-----c---  777

CYTB_SCH  AACCCACTAAACACCCCACCCCATATTAAACCAGAATGATATTTCTTATTCGCCTACGCT  837
CYTB_HSC  ------------------------------------------------------------  837
CYTB_RIN  --t-----c-----a--c--t--c--c-----c--g----c--------t--a-----a  837
CYTB_HIR  --t-----c-----a--c--t--c-----------t-----------c----t--a-----a  837
CYTB_REH  --t-----t-----a--c--t--c------------------c--------t--a-----a  837
CYTB_SHF  --------c-----a--c--t--c--------t-----------c----t--a-----a  837
CYTB_ZIE  --t-----c--t--a--c--t--c--------t--g--g------c----t--a-----a  837
CYTB_PFE  -----t--c-g---t--c--t----------------g--c---c-g--t--------c  837
CYTB_HUN  -----c-----------t--a----------t--g-------tc----------t---  837
CYTB_PUT  --t-----gta-----c--a--c----------g-----c--tc----t--------a  840
CYTB_HHU  ---------gta-----c--a----c-----------------tc----------t--c  840
CYTB_VO1  -----c--ggct-----t--a--c--c-----t--------c---c------a-----a  840
CYTB_KAN  -----c--t--t-----t--------c----------------c--tc----t--------  837
CYTB_KAT  -----tt----t-----t------------t--------c--c-------a-----a  837
CYTB_LAC  -----c---gtt--t-----t-----c--g--t--------c---c-----------a  837
CYTB_AAL  -----ga--gt---------a--c--c--g-----------c---c----t--------a  837

CYTB_SCH  ATTCTACGTTCAATTCCTAATAAACTAGGTGGAGTGTTGGCCCTAGTAGCCTCCATCCTA  897
CYTB_HSC  ------------------------------------------------------------  897
CYTB_RIN  --ct----a-----c--c--c---------a-----ac-a-------cctt---t------  897
CYTB_HIR  --c-----a--------c--c-------a--g--c-a------a-ct-a--t------  897
CYTB_REH  --c-----a--t--------c--------a-----ac-a------a-ct-a--a------  897
CYTB_SHF  --c-----a--------c--------a-----c--a-------cct---g------  897
CYTB_ZIE  --c-----a-----c--c--c--------a-----cc-a--------cct---a------  897
CYTB_PFE  --c-----c--c-----c--c-------c--c--a--a------a-cct--------g  897
CYTB_HUN  --c-----a--c-----------t----a--t--ac-c---------tt---------  897
CYTB_PUT  --c-----c-----c--a--c-----t--a--t--cc-a---t---c---a--ag-a--c  900
CYTB_HHU  --c-----c--c--c--c--c-----t--a--t--ac-a-------c------ag----c  900
CYTB_VO1  --c--c--a-----c--a--c-------a-----cc-a-------cc-----------  900
CYTB_KAN  --c-----c--t-----a--------c--a-----cc-a--t-----ccta-------t  897
CYTB_KAT  -----c--a--c--c--c--c--------g-----cc-a---------ct---------  897
CYTB_LAC  --c-----c--c--------c--------c-----ac-c-----ct--tt---g-----g  897
CYTB_AAL  --c-----a-----c-----c-----c--------ac-a-------c--t----------  897
```

Zu Fig. 2

```
CYTB_SCH  ATCCTAATTTTAATGCCCATACTACACACATCCAAACAACGAGGCATAATATTTCGACCA   957
CYTB_HSC  ------------------------------------------------------------   957
CYTB_RIN  --t--tgc-c----c---c-------------c------------a--------c-----   957
CYTB_HIR  ---t-g--c-c-----tc-t--t----------------ca---g-----c-----   957
CYTB_REH  ---t-g--cc-t--a---c-c--c--t-----t--------ca-t-----g--c--g---   957
CYTB_SHF  ------g-ac-cg-a--tt-c--c--------------t--a---------c-----   957
CYTB_ZIE  ---t--g-ac-tg-a---t-c--c--------t--------a----------c--c---   957
CYTB_PFE  ------gcac-c--c----cc--c----t---a--------a----------c--g--t   957
CYTB_HUN  ---t-ggca--c--t--ac-t--c--------t--g-----ca----------c--g--c   957
CYTB_PUT  --t--tc-cc-t--c---t-c--t--t-a---t--------g-ca----c---c--g---   960
CYTB_HHU  -----ct-cc----c---t-c--c----a--t--------aca----cc--c------   960
CYTB_VO1  g-t---t-cc-------ac-c--------c-----------ctca----cc--c--t---   960
CYTB_KAN  g-t---gcc--c--c--at-c--t--t-tg--t--------ta---g-----c-----c   957
CYTB_KAT  g-a---gcaa-c--t--a--c--c-----c------------a-----g---------   957
CYTB_LAC  g----t--ag-cg-c-----c--c--t--c--t-----------ac-g-cc------c---   957
CYTB_AAL  g----g--ag--g-a--a--t--t-----c--g-----------ac-t-cc--c------   957

CYTB_SCH  CTAAGTCAATGCCTATTCTGAATACTAGTAGCAGACCTCATTACACTAACATGAATTGGA  1017
CYTB_HSC  ------------------------------------------------------------  1017
CYTB_RIN  --c--c---------------gcc--------------ac-g-----c------------  1017
CYTB_HIR  t-t--c---------------ct-----------ac-a-----c-----------  1017
CYTB_REH  t-t---------t----------c--------t-----at-a----------------  1017
CYTB_SHF  a-t--------ta----------ct---g--------ac-a----c-----------  1017
CYTB_ZIE  a-c--c------a----------c--g--------t--at-a-----c-----------  1017
CYTB_PFE  --c--c------g--------c-ct----g------t-ac-g-------------c--c  1017
CYTB_HUN  --t--c---------------c-tt----c--c--t--tc-c--tt------------  1017
CYTB_PUT  --ctca---ac-t--------c-ct--------a-----c-c-tc-----c---g-a---  1020
CYTB_HHU  --ctcc---ac----------c-t--------ca---tc---tc-----c-----c---  1020
CYTB_VO1  --gtca---at---g------g-------c--ca---gc-a-tc----------c--c  1020
CYTB_KAN  a-t------gt----------g-t--c--c----t--tc-c-----c--------c---  1017
CYTB_KAT  -----c-----t---------c-t--------g--t---c-a--c-----------c--t  1017
CYTB_LAC  --c-cc----t-t-------g-cc--g-----g---a-ac-a-tc--t--c--------  1017
CYTB_AAL  gcttca---ct-----------ct----------a-ac-agtg-------------  1017

CYTB_SCH  GGACAACCCGTAGAACACCCGTTCATCATCATCGGCCAACTAGCCTCCATCTTATACTTC  1077
CYTB_HSC  ------------------------------------------------------------  1077
CYTB_RIN  --------a--c--------a-at----c------a--------a--tg--c-------t  1077
CYTB_HIR  -----g--a-t---t----c--t--t--t--t--a--------a--t----------t  1077
CYTB_REH  --c-----a--c---t----c------gct--t------a-t--a--t--ta-g-----t  1077
CYTB_SHF  -----g--a--c--------t-at---g----t--a--------a--t--ta----t---  1077
CYTB_ZIE  -----g--a--c-----t--c-a--t--t--t--a--------a--t--ta----t---  1077
CYTB_PFE  -----g--a--g--------a-a-g-a--t----------g-----a---c-c------  1077
CYTB_HUN  --------a--t--------t--------t-----a---g-c--t--a--------t---  1077
CYTB_PUT  a-c-----a----------a----------t------a----a---c-t-cc------  1080
CYTB_HHU  a-c-----a----------c------------t-----a---a---c---ct------  1080
CYTB_VO1  a-c-----a--t-----a--a--------------a----------ac-aagc------  1080
CYTB_KAN  --c-----a--------------------c---t--a--g---a--tg--c-c------  1077
CYTB_KAT  --c-----t--------t--a-------c-------------------c----t---  1077
CYTB_LAC  --cat-----g--------a-----t-----t--t---a-t-----tg-aa-t-----t  1077
CYTB_AAL  ---at---a------------a-a--t-----t------g----a--ag-ac-t-----t  1077
```

Zu Fig. 2

30

```
CYTB_SCH   CTAATCATTCTAGTATTGATACCAATCACTAGCATCATCGAAAACAACCTATTAAAATGA 1137
CYTB_HSC   ------------------------------------------------------------ 1137
CYTB_RIN   --cc----c-----gc-a-------cgg-cg---ca-----------at--c-------- 1137
CYTB_HIR   t-c--t--c-----cc-t---------t--c----ca--------t-----cc------- 1137
CYTB_REH   --t--t---------c-c---------t-------ca--------t-----c-------- 1137
CYTB_SHF   --t-----------g-a-------g-ag------ct--------t-----cc------- 1137
CYTB_ZIE   --c-----------a-a-------gcag------c---t----------tc-------- 1137
CYTB_PFE   tccc-a-----ca-t--t------c--g-a----c------------t--tc-------- 1137
CYTB_HUN   acc---t-at-ga-cc-a-------cagt----g-t-------------tc-------- 1137
CYTB_PUT   act---t-a---a-cc-ct-c--ct-a-tcg-agc-c-a--------aa--c-c--cctc 1140
CYTB_HHU   acc---c-a--ta-cc-ct-c---c-ca-tcg-a-cac-a--------aa--c-c--c-ac 1140
CYTB_VO1   ac------------c-at-c--t--tg-ag-tg-ac-a--------a---c-----ctc 1140
CYTB_KAN   acc-c---c--ta-tc-t-----cc--g-a---c-a----------aa-cc-c------ 1137
CYTB_KAT   tc--c-c-c---a-cc-a-----c---t-ag----t--t------cgt---c-c------ 1137
CYTB_LAC   act---t-c-----cc-tgcc--cc-gg--g--tgagct-----t--agctc-tg----- 1137
CYTB_AAL   tcct-at-c-----gc-a-ac---c-agtag-ttggc-a--------aa--a----t--- 1137

CYTB_SCH   AGAG .                                                       1141
CYTB_HSC   ---                                                          1140
CYTB_RIN   ---                                                          1140
CYTB_HIR   ---                                                          1140
CYTB_REH   ---                                                          1140
CYTB_SHF   ---                                                          1140
CYTB_ZIE   ---                                                          1140
CYTB_PFE   ---                                                          1140
CYTB_HUN   ---                                                          1140
CYTB_PUT   ta-                                                          1143
CYTB_HHU   ta-                                                          1143
CYTB_VO1   ta-                                                          1143
CYTB_KAN   --                                                           1139
CYTB_KAT   ---                                                          1140
CYTB_LAC   -cct                                                         1141
CYTB_AAL   ta-                                                          1140
```

2u Fig. 2

31

```
HUHN2      GACAAAATTCCATTTCACCCATACTACTCCTTCAAAGACATTCTGGGCTTAACTCTCATA    60
PUTE2      --------c-----c-----c---------ta-----------c--a--tc----aa-----    60
WACHTEL    --t-----c-----c---------------ta-------t--c--a--ac----a--g---    60
ENTE1      --------c-----c---------t------t--------c--a--a--c-tc-----g    60
ENTE3      --------c-----c-----c---t------t--g-----c--a--a--t-tc-----g    60
W-ENTE     -----------------------c----t---------g-----c--a--a--t-tc-----g    60
ENTE2      --t-----c--g--c-----------tt---c-------tg-c--a--g--t-t---t---    60
GANS2      -----------------------t----a--------tact-a--ac-t--ct-a---    60
RIND2      --------c-----c-----c-----ta--a-t--g-----ct-a--ggccctct-ac--    60
SCHWEIN2   -----------------------a-ta-t-----------a--agccttat-t---    60


HUHN2      CTCACCCCATTCCTAACACTAGCCCTATTCTCCCCCAACCTCCTAGGAGACCCAGAAAAC    120
PUTE2      --a------c--a--c---t--a----------a--t------t----------------    120
WACHTEL    --t------------c--c-----------t-----a---t-tt----t-----------    120
ENTE1      --t-----cc-aa--g-----------------a--a-----t-----g-----------    120
ENTE3      --t-----cc--a--g--------------------a--t-----t-----g----------    120
W-ENTE     --t-----cc--a--g--------------------a--t-----t-------------c---    120
ENTE2      --------cc-ga--g--t----------------a--a----------g-----------    120
GANS2      a-------tc-aa--g------------t--a--t--------g-----------c---    120
RIND2      a-tctag-tc-aa--ct------ta------g-a---g-------c-----------t---    120
SCHWEIN2   a-actaatcc-a----tc--t-ta--------a--ag----a--------------c---    120


HUHN2      TTCA    124
PUTE2      --t    123
WACHTEL    --t-    124
ENTE1      ----    124
ENTE3      ----    124
W-ENTE     -a--    124
ENTE2      ----    124
GANS2      -a--    124
RIND2      -a--    124
SCHWEIN2   -a-    123
```

Fig. 3

**EP 1 364 068 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6015664 A

- DE 19629166

**In der Beschreibung aufgeführte Nicht-Patentliteratur**

- **R. MEYER et al.** *Journal of AOAC International,* 1995, vol. 78, 1542-1551
- *Biotechniques,* 1992, vol. 12, 408-411
- *Journal of Molecular Evolution,* 1995, vol. 2, 128-144

- *Proc. Natl. Acad. Sci. USA,* 1989, vol. 86, 6196-6200
- *Appl. Environ. Microbiol.,* 1996, vol. 62, 4060-4065